(19) Europäisches Patentamt
European Patent Office
Office européen des brevets

(11) **EP 3 553 185 B1**

(12) **EUROPEAN PATENT SPECIFICATION**

(45) Date of publication and mention
of the grant of the patent:
**28.07.2021 Bulletin 2021/30**

(51) Int Cl.:
***C12Q 1/6886*** (2018.01)

(21) Application number: **19168813.4**

(22) Date of filing: **12.04.2019**

(54) **METHOD FOR ACQUIRING INFORMATION ON PROGNOSIS OF BREAST CANCER, AND DEVICE FOR DETERMINING PROGNOSIS OF BREAST CANCER**

VERFAHREN ZUR ERFASSUNG VON INFORMATIONEN ZUR PROGNOSE VON BRUSTKREBS UND VORRICHTUNG ZUR BESTIMMUNG DER PROGNOSE VON BRUSTKREBS

PROCÉDÉ D'ACQUISITION D'INFORMATIONS SUR LE PRONOSTIC DU CANCER DU SEIN ET DISPOSITIF POUR DÉTERMINER LE PRONOSTIC DU CANCER DU SEIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.04.2018 JP 2018077986**

(43) Date of publication of application:
**16.10.2019 Bulletin 2019/42**

(73) Proprietors:
• **Osaka University**
**Suita-shi, Osaka 565-0871 (JP)**
• **SYSMEX CORPORATION**
**Kobe-shi**
**Hyogo 651-0073 (JP)**

(72) Inventors:
• **Tsunashima, Ryo**
**Osaka 565-0871 (JP)**
• **Naoi, Yasuto**
**Osaka 565-0871 (JP)**
• **Noguchi, Shinzaburo**
**Osaka 565-0871 (JP)**

(74) Representative: **De Clercq & Partners**
**Edgard Gevaertdreef 10a**
**9830 Sint-Martens-Latem (BE)**

(56) References cited:
**WO-A1-2011/120984    WO-A2-2011/063274**
**US-A1- 2013 178 381**

• **NAOI YASUTO ET AL: "Multi-gene classifiers for prediction of recurrence in breast cancer patients", BREAST CANCER, JAPANESE BREAST CANCER SOCIETY, TOKYO, JP, vol. 23, no. 1, 21 February 2015 (2015-02-21), pages 12-18, XP035590589, ISSN: 1340-6868, DOI: 10.1007/S12282-015-0596-9 [retrieved on 2015-02-21]**
• **SAENGER NICOLE ET AL: "Acid ceramidase is associated with an improved prognosis in both DCIS and invasive breast cancer", MOLECULAR ONCOLOGY, vol. 9, no. 1, January 2015 (2015-01), pages 58-67, XP002793859,**
• **RUCKHAEBERLE EUGEN ET AL: "Microarray analysis of altered sphingolipid metabolism reveals prognostic significance of sphingosine kinase 1 in breast cancer", BREAST CANCER RESEARCH AND TREATMENT, vol. 112, no. 1, November 2008 (2008-11), pages 41-52, XP019639878, ISSN: 0167-6806**

## Description

TECHNICAL FIELD

The present invention relates to a method for acquiring information on prognosis of breast cancer. The present invention also relates to a device for determining prognosis of breast cancer.

BACKGROUND

As multigene assays for recurrence prediction aiming for tailored treatment of breast cancer, Oncotype DX (registered trademark) (Genomic Health, Inc.), MammaPrint (registered trademark) (Agendia), PAM50-ROR (Nanostring Technologies, Inc.), EndoPredict (registered trademark) (Myriad Genetics, Inc.), Curebest (registered trademark) 95GC Breast (Osaka University and Sysmex Corporation: see US Patent Application Publication No. 2011/0263444), and the like are known. These multigene assays are excellent in predicting early recurrence of breast cancer within 5 years after surgery.

As postoperative adjuvant therapy for hormone receptor positive breast cancer, it is standard to administer hormones such as tamoxifen for 5 years after surgery. However, breast cancer may recur also more than 5 years after surgery. Especially, in hormone receptor positive breast cancer, late recurrence more than 5 years after surgery tends to be higher than that in negative breast cancer. In recent years, it has been shown that hormone therapy which administers hormones for 10 years after surgery (extended endocrine treatment) has an effect of reducing risk of recurrence and death, by large-scale clinical trials aTTom and ATLAS. However, long-term administration of hormones has problems of side effects. Also, when extended endocrine treatment is performed for all breast cancer patients, another problem of an increase in medical expenses also arises. Therefore, in clinical sites, a need for a method to predict late recurrence more than 5 years after surgery has rapidly increased in order to decide patients to which extended endocrine treatment is applied. Therefore, attempts have been made to predict late recurrence by the above multigene assays.

SUMMARY OF THE INVENTION

However, the above multigene assays are methods suitable for predicting early recurrence within 5 years after surgery. Early recurrent breast cancer has high cell growth ability, and late recurrent breast cancer has low cell growth ability. Thus, since biological characteristics of early recurrent breast cancer and late recurrent breast cancer differ, prediction accuracy of late recurrence by the multigene assays is limited. Accordingly, it is desirable to develop a method to enable prediction of late recurrence of breast cancer with high accuracy.

The present invention provides a method for acquiring information on prognosis of breast cancer, the method including: a measuring step of measuring expression levels of genes of IL6ST, NPY1R, ELOVL5, OPA1, ASAH1, ALDH6A1 and SYBU in an RNA sample prepared from a biological sample collected from a subject; an analyzing step of analyzing the measured expression levels of genes; and an acquiring step of acquiring information on prognosis of breast cancer based on an analysis result.

The present invention provides a device for determining prognosis of breast cancer, the device including a processor, and a computer containing a memory under control of the processor, wherein the memory is recorded with a computer program for causing the computer to execute the steps of: acquiring information on expression levels of genes of IL6ST, NPY1R, ELOVL5, OPA1, ASAH1, ALDH6A1 and SYBU in an RNA sample prepared from a biological sample collected from a subject; determining prognosis of breast cancer based on the information on the expression levels of genes; and outputting a determination result.

The present invention provides a computer program for determining prognosis of breast cancer recorded on a computer readable medium, the computer program causing a computer to execute the steps of: acquiring information on expression levels of genes of IL6ST, NPY1R, ELOVL5, OPA1, ASAH1, ALDH6A1 and SYBU in an RNA sample prepared from a biological sample collected from a subject; determining prognosis of breast cancer based on the information on the expression levels of genes; and outputting a determination result.

The present invention enables prediction of prognosis of breast cancer in a subject, especially, late recurrence after surgery with high accuracy.

BRIEF DESCRIPTION OF THE DRAWINGS

Fig. 1 is a decision tree of a treatment policy for patients with estrogen receptor (ER)-positive, locally advanced breast cancer.

Fig. 2 is a decision tree of a treatment policy for patients with ER-positive early breast cancer (pN0: no lymph node metastasis).

Fig. 3 is a decision tree of a treatment policy for patients with ER-positive early breast cancer (pN1-3: with lymph node metastasis).

Fig. 4 is a decision tree of a treatment policy for patients with ER-positive early breast cancer (pN1: with lymph node metastasis).

Fig. 5 is a schematic diagram showing an example of a device for determining prognosis of breast cancer.

Fig. 6 is a block diagram showing a hardware configuration of the device for determining prognosis of breast cancer.

Fig. 7 is a flowchart for determination using the device for determining prognosis of breast cancer.

Fig. 8 is a graph showing a relationship between the number of probe sets and accuracy or negative predictive value (NPV).

Fig. 9A is a graph showing a relationship between period after surgery and distant recurrence-free survival rate in a training set.

Fig. 9B is a graph showing a relationship between period after surgery and distant recurrence-free survival rate in a validation set.

## DETAILED DESCRIPTION OF THE PREFERRED EMBODIMENTS

A method for acquiring information on prognosis of breast cancer of the present embodiment (hereinafter, also simply referred to as "method") enables acquisition of information on prognosis of breast cancer of a subject, by measuring and analyzing expression levels of predetermined genes. As the information on prognosis of breast cancer, information on risk of recurrence after surgery, especially, information on risk of late recurrence after surgery can be acquired. More specifically, according to the method of the present embodiment, the risk of recurrence after surgery in a subject, especially, whether the risk of late recurrence after surgery is high or low can be accurately predicted. In the present specification, the term "late recurrence" means recurrence more than 5 years after surgery. The phrase "more than 5 years after surgery" refers to a day after 5 years have elapsed from a day of surgery (that is, it does not include a day of 5 years after surgery). The method of the present embodiment is especially suitable for predicting risk of recurrence more than 5 years within 10 years after surgery.

By providing information obtained by the method of the present embodiment to a person who decides diagnosis and treatment policy (for example, a doctor or the like), it is possible to assist diagnosis of recurrence risk of breast cancer. Based on the information obtained by the method of the present embodiment, it is possible to decide necessity of hormone therapy over a long period of 10 years, so that reduction of side effects and suppression of medical expenses are expected.

In the method of the present embodiment, first, expression levels of genes of IL6ST, NPY1R, ELOVL5, OPA1, ASAH1, ALDH6A1 and SYBU in an RNA sample prepared from a biological sample collected from a subject are measured. Hereinafter, these seven genes are also referred to as "first gene group".

In a preferred embodiment, in addition to the above seven genes, an expression level of at least one gene selected from CALB2, ASAP2, RAB5C, PTP4A2, ABCC10, OXTR, HSPA2, SMURF2, SLC7A8, RALA, ADRA2A, MYCBP, CX3CR1, ERCC1, DNAJA3, NINJ1, C4orf43, IFI35, ZNF688, SNX1, CREBL2, HPN, NME3, STS, KLF7, PDHB, NKX3-1, DEXI, GSTM3 and LCMT1 is further measured. Hereinafter, these 30 genes are also referred to as "second gene group". In the present embodiment, it is particularly preferable to measure the expression levels of all 37 genes contained in the first and second gene groups.

The subject is a breast cancer patient. It is known that ER-positive breast cancer patients tend to recur more than 5 years after surgery, compared with ER-negative breast cancer patients. Accordingly, an ER-positive breast cancer patient is preferable as the breast cancer patient. The subject may be undergoing preoperative adjuvant therapy as long as a biological sample containing breast cancer cells can be collected. Examples of the preoperative adjuvant therapy include administration of hormones, administration of anticancer agents, and combinations thereof. Examples of the hormones include tamoxifen, toremifene, leuprorelin acetate, goserelin acetate, anastrozole, fulvestrant, and the like. Examples of the anticancer agents include adriamycin, epirubicin, paclitaxel, docetaxel, cyclophosphamide, methotrexate, fluorouracil, and the like.

Since the method of the present embodiment can predict recurrence risk more than 5 years after surgery, the subject may be a subject who did not have recurrence of breast cancer, for example, for 5 years after surgery. The subject may be a subject whose risk of early recurrence of breast cancer has been determined to be low by a prognosis prediction method publicly known in the art. As such a prognosis prediction method, a method capable of predicting early recurrence of breast cancer is preferable. Examples include multigene assays such as Oncotype DX (registered trademark), MammaPrint (registered trademark), PAM50-ROR, EndoPredict (registered trademark) and Curebest (registered trademark) 95GC Breast. The term "early recurrence" refers to recurrence within 5 years after surgery. In the present specification, the phrases "within 5 years after surgery" and "for 5 years after surgery" refer to a day until 5 years have elapsed from the day of surgery (that is, they include the day of 5 years after surgery).

As described above, ER-positive breast cancer tends to recur late, and ER-negative breast cancer tends to recur early. One of causes of breast cancer recurrence includes micrometastasis. The present inventors have searched for marker genes for the purpose of establishing an inspection method for predicting recurrence time, when breast cancer remains as a micrometastasis in the body after surgery. Specifically, patients with breast cancer recurrence have been divided into an early recurrence group and a late recurrence group according to recurrence time, and differences in gene expression levels between the two groups have been comprehensively analyzed. As a result, the present inventors have identified the above 37 genes as genes associated with late recurrence of breast cancer. Thus, in a preferred embodiment, the subject is an ER-positive breast cancer patient, and expression levels of genes of IL6ST, NPY1R, ELOVL5, OPA1, ASAH1, ALDH6A1, SYBU, CALB2, ASAP2, RAB5C, PTP4A2, ABCC10, OXTR, HSPA2, SMURF2, SLC7A8, RALA, ADRA2A, MYCBP, CX3CR1, ERCC1, DNAJA3, NINJ1, C4orf43, IFI35, ZNF688, SNX1, CREBL2, HPN, NME3, STS, KLF7, PDHB, NKX3-1, DEXI, GSTM3 and LCMT1 in an RNA sample prepared from a biological sample collected from the subject are measured.

In general, colorectal cancer, lung cancer, ER-negative breast cancer and the like are known to have a fast growth rate among cancers. When such cancer with a fast growth rate exists in the body as a micrometastasis which cannot be pointed out by image inspection, it is considered that the cancer will recur within 5 years after surgery. However, no one knows whether or not there is such a micrometastasis at the time of surgery. While cure may be achieved by effects of surgery, preoperative or postoperative drug therapy and the like, the therapeutic effects cannot be examined even by assays such as microarray. Therefore, in clinical practice, when a patient has no recurrence for 5 years, it is common to finish follow-up of the patient, considering that cancer has been cured. However, ER-positive breast cancer has a slower growth rate than ER-negative breast cancer, and since hormone therapy is effective, cases recurring more than 5 years after surgery are seen. Therefore, it is dangerous to consider that cancer has been cured in ER-positive breast cancer even when the patient has no recurrence for 5 years after surgery. On the other hand, there are not a few cases of ER-positive breast cancer recurred within 5 years after surgery. The present inventors have considered that, among ER-positive breast cancers, there exists breast cancer with a fast growth rate that recurs within 5 years after surgery when there is micrometastasis at the time of surgery by about 30 to 50%, as with ER-negative breast cancer. In a case where such breast cancer with a fast growth rate and breast cancer with a slow growth rate can be accurately found in an inspection, it can be considered that breast cancer has been cured when ER-positive breast cancer with a fast growth rate does not recur for 5 years after surgery. When breast cancer is cured, administration of hormones more than 5 years after surgery is unnecessary. In the present embodiment, the expression levels of all the above 37 genes in an RNA sample prepared from a biological sample collected from an ER-positive breast cancer patient are measured and analyzed, whereby risk of late recurrence of the patient can be accurately predicted. When ER-positive breast cancer does not recur for 5 years after surgery in a patient whose risk of late recurrence has been determined to be low, extended endocrine treatment can be safely omitted.

The biological sample is not particularly limited as long as it contains breast cancer cells of the subject. Examples thereof include cells collected by fine-needle aspiration, tissues collected by surgery or biopsy, and the like. A formalin-fixed paraffin-embedded (FFPE) specimen prepared from cells or tissues collected from the subject may be used as the biological sample. In a preferred embodiment, the biological sample is a cell or tissue collected from a subject not yet undergoing treatment for breast cancer, or a breast cancer patient undergoing only preoperative adjuvant therapy with hormones, or an FFPE specimen thereof.

The RNA sample contains RNA extracted from the biological sample or a nucleic acid derived from the RNA, and is a sample to be subjected to measurement of the gene expression levels. A method for extracting RNA from the biological sample is publicly known per se. When the biological sample is a cell or a tissue, extraction of RNA can be performed, for example, as follows. First, the biological sample is mixed with a solubilizing solution containing guanidine thiocyanate and a surfactant. Physical treatment (stirring, homogenizing, ultrasonic disruption, etc.) is performed on the resulting mixed solution to release RNA contained in the biological sample into the mixed solution. Thus, RNA can be extracted from the biological sample. The extracted RNA may be purified. For example, RNA can be purified by centrifuging a mixed solution containing RNA to collect a supernatant, and extracting the supernatant with phenol/chloroform. Extraction and purification of RNA from the biological sample may be performed using a commercially available RNA extraction kit.

When the biological sample is an FFPE specimen, extraction of RNA can be performed, for example, as follows. First, xylene is added to the FFPE specimen and deparaffinization treatment is performed. The deparaffinized specimen is immersed in ethanol to make it hydrophilic. By treating the hydrophilized specimen with a protease to release formalin-crosslinked nucleic acid, RNA can be extracted from the specimen. Extraction and purification of RNA from the FFPE specimen may be performed by using a commercially available nucleic acid extraction kit for FFPE specimen.

In the present embodiment, RNA extracted from the biological sample and purified may be used as the RNA sample. Alternatively, cDNA obtained by reverse transcribing mRNA contained in the RNA may be used, or cRNA obtained by in vitro transcription (IVT) amplification of the cDNA may be used as the RNA sample. A method for acquiring cDNA and cRNA is publicly known per se.

In the present specification, the term "expression level of a gene" means the amount of mRNA transcribed from a gene or the amount of substance reflecting the amount of mRNA. Examples of the substance reflecting the amount of mRNA include cDNA, cRNA, and the like. Since the amount of mRNA transcribed from a gene is often very small, it is preferable to measure the amount of cDNA or cRNA. The expression level of a gene may be represented by either concentration, copy number, or a measured value indicating concentration or copy number.

The nucleotide sequence of each of the above genes is publicly known per se. Information on these nucleotide sequences can be obtained, for example, from a publicly known database such as a database (http://www.ncbi.nlm.nih.gov/) provided by the National Center for Biotechnology Information (NCBI) of the United States National Library of Medicine. Table 1-1 and Table 1-2 show UniGene IDs, GenBank accession numbers and probe set IDs of each gene. The probe set IDs shown in Table 1-1 and Table 1-2 are numbers for specifying a probe set loaded on GeneChip (registered trademark) Human Genome U133 Plus 2.0 Array (Affymetrix, Inc.). Each probe set contains 11 to 20 probes. Information on nucleotide sequences of probes included in a probe set specified by each probe set ID can be obtained from Affymetrix's web page (http://www.affymetrix.com/analysis/index.affx) and the like. The "UniGene ID" is ID number of UniGene which is a database published by NCBI. The "GenBank accession number" is an accession number of public database GenBank used for designing a sequence of each probe of microarray GeneChip (registered trademark) manufactured by Affymetrix, Inc. The "gene number" is a number assigned to a gene corresponding to each probe set ID.

[Table 1]

Table 1-1

| Gene number | Probe set ID | Gene symbol | UniGene ID | GenBank accession number |
|---|---|---|---|---|
| 1 | 212196_at | IL6ST | Hs.532082 | AW242916 |
| 2 | 205440_s_at | NPY1R | Hs.519057 | NM_000909 |
| 3 | 208788_at | ELOVL5 | Hs.520189 | AL136939 |
| 4 | 214306_at | OPA1 | Hs.594504 | AA209332 |
| 5 | 212195_at | IL6ST | Hs.532082 | AL049265 |
| 6 | 210980_s_at | ASAH1 | Hs.527412 | U47674 |
| 7 | 204864_s_at | IL6ST | Hs.532082 | NM_002184 |
| 8 | 221590_s_at | ALDH6A1 | Hs.293970 | AF130089 |
| 9 | 218692_at | SYBU | Hs.390738 | NM_017786 |

[Table 2]

Table 1-2

| Gene number | Probe set ID | Gene symbol | UniGene ID | GenBank accession number |
|---|---|---|---|---|
| 10 | 205428_s_at | CALB2 | Hs.106857 | NM_001740 |
| 11 | 206414_s_at | ASAP2 | Hs.555902 | NM_003887 |
| 12 | 201156_s_at | RAB5C | Hs.650382 | AF141304 |
| 13 | 216988_s_at | PTP4A2 | Hs.470477 | L48722 |
| 14 | 213485_s_at | ABCC10 | Hs.55879 | AK000002 |
| 15 | 206825_at | OXTR | Hs.2820 | NM_000916 |
| 16 | 211538_s_at | HSPA2 | Hs.432648 | U56725 |
| 17 | 205596_s_at | SMURF2 | Hs.515011 | AY014180 |

(continued)

| Gene number | Probe set ID | Gene symbol | UniGene ID | GenBank accession number |
|---|---|---|---|---|
| 18 | 202752_x_at | SLC7A8 | Hs.596643 | NM_012244 |
| 19 | 214435_x_at | RALA | Hs.6906 | NM_005402 |
| 20 | 209869_at | ADRA2A | Hs.249159 | AF284095 |
| 21 | 203360_s_at | MYCBP | Hs.591506 | D50692 |
| 22 | 205898_at | CX3CR1 | Hs.78913 | U20350 |
| 23 | 203720_s_at | ERCC1 | Hs.435981 | NM_001983 |
| 24 | 205963_s_at | DNAJA3 | Hs.459779 | NM_005147 |
| 25 | 211000_s_at | IL6ST | Hs.532082 | AB015706 |
| 26 | 203045_at | NINJ1 | Hs.494457 | NM_004148 |
| 27 | 218513_at | C4orf43 | Hs.267446 | NM_018352 |
| 28 | 209417_s_at | IFI35 | Hs.632258 | BC001356 |
| 29 | 213527_s_at | ZNF688 | Hs.301463 | AI095896 |
| 30 | 201716_at | SNX1 | Hs.188634 | NM_003099 |
| 31 | 201988_s_at | CREBL2 | Hs.591156 | BF438056 |
| 32 | 204934_s_at | HPN | Hs.182385 | NM_002151 |
| 33 | 204862_s_at | NME3 | Hs.514065 | NM_002513 |
| 34 | 203769_s_at | STS | Hs.522578 | NM_000351 |
| 35 | 204334_at | KLF7 | Hs.471221 | AA488672 |
| 36 | 208911_s_at | PDHB | Hs.161357 | M34055 |
| 37 | 204863_s_at | IL6ST | Hs.532082 | BE856546 |
| 38 | 209706_at | NKX3-1 | Hs.55999 | AF247704 |
| 39 | 203733_at | DEXI | Hs.592051 | NM_014015 |
| 40 | 213702_x_at | ASAH1 | Hs.527412 | AI934569 |
| 41 | 202554_s_at | GSTM3 | Hs.2006 | AL527430 |
| 42 | 221515_s_at | LCMT1 | Hs.337730 | BC001214 |

A method for measuring the expression levels of genes is not particularly limited, and can be appropriately selected from publicly known measurement methods. Examples of the method include microarrays, quantitative RT-PCR, quantitative PCR, and the like. In the present embodiment, it is preferable to measure the expression levels of genes using a microarray. In this case, it is more preferable to measure expression levels of genes using probe sets specified by the probe set IDs shown in Table 1-1 and Table 1-2. For example, when the expression levels of seven genes of the first gene group are measured, it is preferable to use probe sets specified by nine probe set IDs shown in Table 1-1. When the expression level of at least one gene selected from the second gene group is further measured in addition to the above seven genes, it is preferable to use the probe sets specified by nine probe set IDs shown in Table 1-1 and a probe set specified by a probe set ID corresponding to the gene to be measured shown in Table 1-2. When the expression levels of all 37 genes contained in the first and second gene groups are measured, it is preferable to use the probe sets specified by 42 probe set IDs shown in Table 1-1 and Table 1-2.

The microarray is not particularly limited as long as it is a chip in which a probe capable of specifically hybridizing to mRNA transcribed from each of the above genes, or cDNA or cRNA derived from the mRNA (hereinafter also referred to as "target nucleic acid") is immobilized on a suitable substrate. The probe can be appropriately designed based on the nucleotide sequence of each of the above genes. The length of the probe may be 10 to 50 nucleotides. The microarray itself can be prepared by a publicly known method. In the present embodiment, it is preferable to use a microarray loading probe sets specified by the probe set IDs shown in Table 1-1 and Table 1-2. A commercially available microarray may be used.

As used herein, the phrase "capable of specifically hybridizing" means that a probe can hybridize to a target nucleic acid under stringent conditions. The term "stringent conditions" means conditions commonly used by those skilled in the art under which hybridization of polynucleotides is performed. It is known that the stringency of conditions under which hybridization is performed is a function of temperature, salt concentration of hybridization buffer, probe length, GC content of nucleotide sequence of probe and concentration of chaotropic agent in hybridization buffer, and those skilled in the art can suitably set in consideration of these conditions. As the stringent conditions, conditions described in, for example, Molecular Cloning: A Laboratory Manual (second edition) (Sambrook, J. et al., Cold Spring Harbor Laboratory Press, New York (1989)) and the like can be used.

When the microarray is used, it is preferable that the target nucleic acid is labeled with a publicly known labeling substance. By labeling the target nucleic acid, it is easy to measure a signal from the probe on the microarray. In the present embodiment, while mRNA extracted from the biological sample may be labeled, it is preferable to label cDNA or cRNA derived from the mRNA. Examples of the labeling substance include fluorescent substances, hapten such as biotin, radioactive substances, and the like. Examples of the fluorescent substance include Cy3, Cy5, FITC, Alexa Fluor (trademark), and the like.

In measurement by the microarray, the expression level of gene is obtained as a signal from the probe, such as fluorescence intensity, luminescence intensity or current amount. The signal can be detected by a scanner included in a microarray analyzer. Examples of the scanner include GeneChip (registered trademark) Scanner3000 7G (Affymetrix, Inc.), Illumina (registered trademark) BeadArray Reader (Illumina, Inc.), and the like.

Next, in the method of the present embodiment, the measured gene expression levels are analyzed. In the present embodiment, measurement data (raw data) of gene expression levels may be normalized by a publicly known statistical algorithm for analysis. Examples of such a statistical algorithm include RMA, MAS5, PLIER, and the like. RMA, MAS5 and PLIER can be used, for example, in Affymetrix Expression Console (trademark) software (manufactured by Affymetrix, Inc.).

The gene expression levels can be analyzed by a publicly known method such as a classification method, a scoring method, or a hierarchical cluster analysis. Examples of the classification method include diagonal linear discriminant analysis (DLDA), between-group analysis (BGA) (see Culhane A.C. et al., Between-group analysis of microarray data, Bioinformatics, 2002, vol. 18, p. 1600-1608), support vector machine (SVM), k nearest neighbor classification (kNN), decision tree, random forest, neural net, and the like.

Hierarchical cluster analysis can be performed, for example, as follows. Using data on expression levels of a specimen (RNA sample) derived from a subject, data on expression levels in a group of specimens known to have good prognosis, data on expression levels in a group of specimens known to recur late, a distance indicating similarity between specimens is calculated based on the expression levels. Based on this distance, various clusters are formed. Analysis is performed by integrating clusters and creating tree diagrams. Examples of the distance include Spearman rank correlation coefficient, Euclidean distance, and the like. Integration of clusters can be performed, for example, by ward method, furthest neighbor method, distance-between-centroids method, and the like. Among them, the Spearman rank correlation coefficient and the ward method are preferably used.

Examples of the scoring method include principal component analysis, multiple regression analysis, logistic regression analysis, Partial Least Square, and the like. When the gene expression levels are analyzed using the scoring method, scoring is performed so as to classify a score of a specimen predicted to have a good prognosis and a score of a specimen predicted to have a poor prognosis, based on the expression levels.

When the gene expression levels are analyzed by DLDA classification method, a discriminant constructed using DLDA can be used. For example, when expression levels of genes are measured using the probe sets specified by the probe set IDs shown in Table 1, the discriminant represented by the following formula (I) is shown as the discriminant.

$$D = \Sigma_i(w_i \times y_i) - (-4.763756453) \cdots (I)$$

wherein i represents a number assigned to each gene shown in Table 2-1 and Table 2-2, $w_i$ represents a weighting factor of gene numbered i shown in Table 2-1 and Table 2-2, $y_i$ represents a standardized expression level of gene according to a formula represented by formula (II):

$$y_i = x_i - m_i \cdots (II)$$

wherein $x_i$ represents an expression level of gene numbered i shown in Table 2-1 and Table 2-2, and $m_i$ represents a mean value of expression levels of genes numbered i shown in Table 2-1 and Table 2-2 over specimens,

and $\Sigma_i$ represents a sum total over the genes.

[Table 3]

Table 2-1

| Gene number | Probe set ID | Gene symbol | Weighting factor |
|---|---|---|---|
| 1 | 212196_at | IL6ST | 1.43235997 |
| 2 | 205440_s_at | NPY1R | 0.450736337 |
| 3 | 208788_at | ELOVL5 | 1.793041948 |
| 4 | 214306_at | OPA1 | -2.545950461 |
| 5 | 212195_at | IL6ST | 1.539087017 |
| 6 | 210980_s_at | ASAH1 | 1.539921375 |
| 7 | 204864_s_at | IL6ST | 1.151743979 |
| 8 | 221590_s_at | ALDH6A1 | 1.837908119 |
| 9 | 218692_at | SYBU | 1.149172541 |

[Table 4]

Table 2-2

| Gene number | Probe set ID | Gene symbol | Weighting factor |
|---|---|---|---|
| 10 | 205428_s_at | CALB2 | -1.179415269 |
| 11 | 206414_s_at | ASAP2 | -2.323532179 |
| 12 | 201156_s_at | RAB5C | 2.316847761 |
| 13 | 216988_s_at | PTP4A2 | 2.002072892 |
| 14 | 213485_s_at | ABCC10 | -2.306701292 |
| 15 | 206825_at | OXTR | -1.1298499 |
| 16 | 211538_s_at | HSPA2 | 0.964294299 |
| 17 | 205596_s_at | SMURF2 | -2.109236485 |
| 18 | 202752_x_at | SLC7A8 | 1.363057605 |
| 19 | 214435_x_at | RALA | -2.288374092 |
| 20 | 209869_at | ADRA2A | 1.377444437 |
| 21 | 203360_s_at | MYCBP | 1.688897659 |
| 22 | 205898_at | CX3CR1 | 0.740592577 |
| 23 | 203720_s_at | ERCC1 | 1.658860215 |
| 24 | 205963_s_at | DNAJA3 | 2.534304027 |
| 25 | 211000_s_at | IL6ST | 0.971312607 |
| 26 | 203045_at | NINJ1 | 1.889380552 |
| 27 | 218513_at | C4orf43 | 1.429869188 |
| 28 | 209417_s_at | IFI35 | 1.323441517 |

(continued)

| Gene number | Probe set ID | Gene symbol | Weighting factor |
|---|---|---|---|
| 29 | 213527_s_at | ZNF688 | 2.158913805 |
| 30 | 201716_at | SNX1 | 2.2492475 |
| 31 | 201988_s_at | CREBL2 | 1.921069642 |
| 32 | 204934_s_at | HPN | 1.115597062 |
| 33 | 204862_s_at | NME3 | 1.661682832 |
| 34 | 203769_s_at | STS | -1.392023997 |
| 35 | 204334_at | KLF7 | -1.662199312 |
| 36 | 208911_s_at | PDHB | 2.071027409 |
| 37 | 204863_s_at | IL6ST | 0.825924727 |
| 38 | 209706_at | NKX3-1 | 0.611126615 |
| 39 | 203733_at | DEXI | 1.847912036 |
| 40 | 213702_x_at | ASAH1 | 1.575733773 |
| 41 | 202554_s_at | GSTM3 | 0.648154701 |
| 42 | 221515_s_at | LCMT1 | 1.7259299 |

$m_i$ in the formula (II) can be acquired as follows. For example, when the method according to the present embodiment is performed on each of a plurality of subjects in one cohort, first, expression levels of genes are measured using the probe sets shown in Table 2-1 and Table 2-2, for specimens (RNA samples) derived from each of the plurality of subjects. From the measured expression levels of genes, a mean value in the specimens of the plurality of subjects is calculated. The obtained mean value of the expression levels of genes can be used as $m_i$.

Alternatively, with respect to a plurality of breast cancer patients in the same facility, expression levels of the genes shown in Table 2-1 and Table 2-2 are measured and data are accumulated, and a mean value may be calculated for the expression levels of the genes of those patients. The mean value of the expression levels of the genes thus obtained can be used as $m_i$ in the formula (II) when the method of the present embodiment is performed on a predetermined subject in the facility.

When the expression levels are analyzed using the discriminant represented by the formula (I), the value of the expression level in the specimen is assigned to $x_i$ (i = 1, 2, ..., 42) of the formula (II) in sequence, to calculate solution D. When the solution D is a positive value, it can be determined that risk of late recurrence of the subject is high. When the solution D is zero or a negative value, it can be determined that the risk of late recurrence of the subject is low. For a subject whose risk of late recurrence has been determined to be high, it may be decided to administer hormones until 10 years after surgery. For a subject whose risk of late recurrence has been determined to be low, it may be decided to administer hormones until 5 years after surgery.

The above 42 probe sets correspond to genes whose expression levels are significantly different between the late recurrence group and the early recurrence group. Therefore, when it has been determined that the risk of late recurrence is low based on the expression levels of genes measured by the probe sets, it means the same as it has been determined that the risk of early recurrence is high. Accordingly, in the present embodiment, a determination result that the risk of late recurrence is low may be rephrased as a determination result that the risk of early recurrence is high. That is, by performing the method of the present embodiment immediately after surgery, it can be predicted whether the risk of late recurrence is high or the risk of early recurrence is high for a subject. When breast cancer does not recur for 5 years after surgery in the subject whose risk of late recurrence has been determined to be low (i.e., the subject whose risk of early recurrence has been determined to be high), it may be considered that breast cancer has been cured.

In the present embodiment, collection of a biological sample, measurement of gene expression levels, and analysis of the gene expression levels may be performed at substantially the same time. In another embodiment, collection of a biological sample and measurement and analysis of gene expression levels may be performed at times apart by a predetermined period. For example, cells or tissues collected from a subject are stored as an FFPE specimen, and after a predetermined period (for example, 5 years after surgery) has elapsed, measurement and analysis of expression levels of the genes may be performed using an RNA sample prepared from the FFPE specimen. Alternatively, collection of a biological sample, measurement of gene expression levels, and analysis of the gene expression levels may be performed at times apart by a predetermined period. For example, an RNA sample is prepared from cells or tissues collected from a subject, expression levels of the genes are measured, measurement data is stored, and after a predetermined period (for example, 5 years after surgery) has elapsed, analysis of the expression levels may be performed using the measurement data.

Since the method of the present embodiment can accurately predict the risk of late recurrence of breast cancer, it is possible to predict the recurrence risk within 10 years after surgery, by combining the method of the present embodiment with a publicly known method for predicting the risk of early recurrence of breast cancer. For example, the method of the present embodiment and the method for predicting the risk of early recurrence of breast cancer may be performed at the same time. Alternatively, first, the method for predicting the risk of early recurrence of breast cancer is performed, and when it has been determined that the recurrence risk within 5 years after surgery is low, the method of the present embodiment may be performed. Alternatively, when it is determined that the recurrence risk within 5 years after surgery is low by the method for predicting the risk of early recurrence of breast cancer, and the subject does not actually have recurrence of breast cancer for 5 years after surgery, the method of the present embodiment may be performed at 5 years after surgery. In the present embodiment, when it has been determined that the recurrence risk within 5 years after surgery is high by the method for predicting the risk of early recurrence of breast cancer, the method of the present embodiment may be further performed.

When the method of the present embodiment is combined with a publicly known method for predicting the risk of early recurrence of breast cancer, the expression levels of genes used in the method of the present embodiment and the expression levels of genes used in the method for predicting the risk of early recurrence may be acquired by a single measurement, for example, with a microarray. In this case, all the measured expression levels of genes may be simultaneously analyzed. Alternatively, the expression levels of genes used in the method for predicting the risk of early recurrence are analyzed, then the expression levels of genes used in the method of the present embodiment may be analyzed.

When a part of cells or tissues collected from a subject is stored as an FFPE specimen, first, from an RNA sample prepared from the cells or tissues, measurement and analysis of expression levels of the genes used in the method for predicting the risk of early recurrence are performed. After that (for example, after 5 years), from the RNA sample prepared from the FFPE specimen, the expression levels of genes used in the method of the present embodiment may be measured and analyzed.

A publicly known method for predicting the risk of early recurrence of breast cancer is not particularly limited, and for example, the method described in Patent Document 1 is preferable. In the present embodiment, the subject may be a subject whose risk of early recurrence of breast cancer has been determined to be low, based on gene expression levels measured using probe sets specified by 95 probe set IDs shown in Table 3-1 and Table 3-2.

[Table 5]

Table 3-1

| Number | Probe set ID | Gene symbol | UniGene ID | GenBank accession number |
|---|---|---|---|---|
| 1 | 219306_at | KIF15 | Hs.646856 | NM_020242 |
| 2 | 218585_s_at | DTL | Hs.656473 | NM_016448 |
| 3 | 221677_s_at | DONSON | Hs.436341 | AF232674 |
| 4 | 201088_at | KPNA2 | Hs.594238 | NM_002266 |
| 5 | 209034_at | PNRC1 | Hs.75969 | AF279899 |
| 6 | 202610_s_at | MED14 | Hs.407604 | AF135802 |
| 7 | 218906_x_at | KLC2 | Hs.280792 | NM_022822 |
| 8 | 212723_at | JMJD6 | Hs.514505 | AK021780 |
| 9 | 222231_s_at | LRRC59 | Hs.370927 | AK025328 |
| 10 | 208838_at | CAND1 | Hs.546407 | AB020636 |
| 11 | 218039_at | NUSAP1 | Hs.615092 | NM_016359 |
| 12 | 209472_at | CCBL2 | Hs.481898 | BC000819 |

(continued)

| Number | Probe set ID | Gene symbol | UniGene ID | GenBank accession number |
|---|---|---|---|---|
| 13 | 212898_at | KIAA0406 | Hs.655481 | AB007866 |
| 14 | 202620_s_at | PLOD2 | Hs.477866 | NM_000935 |
| 15 | 201059_at | CTTN | Hs.596164 | NM_005231 |
| 16 | 201841_s_at | HSPB1 | Hs.520973 | NM_001540 |
| 17 | 203755_at | BUB1B | Hs.631699 | NM_001211 |
| 18 | 211750_x_at | TUBA1C | Hs.719091 | BC005946 |
| 19 | 38158_at | ESPL1 | Hs.153479 | D79987 |
| 20 | 204709_s_at | KIF23 | Hs.270845 | NM_004856 |
| 21 | 201589_at | SMC1A | Hs.211602 | D80000 |
| 22 | 218460_at | HEATR2 | Hs.535896 | NM_017802 |
| 23 | 207430_s_at | MSMB | Hs.255462 | NM_002443 |
| 24 | 212139_at | GCN1L1 | Hs.298716 | D86973 |
| 25 | 211596_s_at | LRIG1 | Hs.518055 | AB050468 |
| 26 | 212160_at | XPOT | Hs.85951 | AI984005 |
| 27 | 219238_at | PIGV | Hs.259605 | NM_017837 |
| 28 | 203432_at | TMPO | Hs.11355 | AW272611 |
| 29 | 201377_at | UBAP2L | Hs.490551 | NM_014847 |
| 30 | 218875_s_at | FBXO5 | Hs.520506 | NM_012177 |
| 31 | 221922_at | GPSM2 | Hs.584901 | AW195581 |
| 32 | 218727_at | SLC38A7 | Hs.10499 | NM_018231 |
| 33 | 207469_s_at | PIR | Hs.495728 | NM_003662 |
| 34 | 218483_s_at | C11orf60 | Hs.533738 | NM_020153 |
| 35 | 204641_at | NEK2 | Hs.153704 | NM_002497 |
| 36 | 219502_at | NEIL3 | Hs.405467 | NM_018248 |
| 37 | 209054_s_at | WHSC1 | Hs.113876 | AF083389 |
| 38 | 220318_at | EPN3 | Hs.670090 | NM_017957 |
| 39 | 210297_s_at | MSMB | Hs.255462 | U22178 |
| 40 | 209186_at | ATP2A2 | Hs.506759 | M23114 |
| 41 | 219787_s_at | ECT2 | Hs.518299 | NM_018098 |
| 42 | 45633_at | GINS3 | Hs.47125 | AI421812 |
| 43 | 200848_at | AHCYL1 | Hs.705418 | AA479488 |
| 44 | 200822_x_at | TPI1 | Hs.524219 | NM_000365 |
| 45 | 211072_x_at | TUBA1B | Hs.719075 | BC006481 |
| 46 | 200811_at | CIRBP | Hs.634522 | NM_001280 |
| 47 | 202864_s_at | SP100 | Hs.369056 | NM_003113 |
| 48 | 202154_x_at | TUBB3 | Hs.511743 | NM_006086 |
| 49 | 213152_s_at | SFRS2B | Hs.476680 | AI343248 |
| 50 | 209368_at | EPHX2 | Hs.212088 | AF233336 |

[Table 6]

Table 3-2

| Number | Probe set ID | Gene symbol | UniGene ID | GenBank accession number |
|---|---|---|---|---|
| 51 | 211058_x_at | TUBA1B | Hs.719075 | BC006379 |
| 52 | 209251_x_at | TUBA1C | Hs.719091 | BC004949 |
| 53 | 213646_x_at | TUBA1B | Hs.719075 | BE300252 |
| 54 | 204540_at | EEF1A2 | Hs.433839 | NM_001958 |
| 55 | 202026_at | SDHD | Hs.719164 | NM_003002 |
| 56 | 201090_x_at | TUBA1B | Hs.719075 | NM_006082 |
| 57 | 213119_at | SLC36A1 | Hs.269004 | AW058600 |
| 58 | 217840_at | DDX41 | Hs.484288 | NM_016222 |
| 59 | 206559_x_at | EEF1A1 | --- | NM_001403 |
| 60 | 202066_at | PPFIA1 | Hs.530749 | AA195259 |
| 61 | 203108_at | GPRC5A | Hs.631733 | NM_003979 |
| 62 | 218697_at | NCKIPSD | Hs.655006 | NM_016453 |
| 63 | 222039_at | KIF18B | Hs.135094 | AA292789 |
| 64 | 202069_s_at | IDH3A | Hs.591110 | AI826060 |
| 65 | 203362_s_at | MAD2L1 | Hs.591697 | NM_002358 |
| 66 | 202666_s_at | ACTL6A | Hs.435326 | NM_004301 |
| 67 | 204892_x_at | EEF1A1 | Hs.520703 | NM_001402 |
| 68 | 205682_x_at | APOM | Hs.534468 | NM_019101 |
| 69 | 209714_s_at | CDKN3 | Hs.84113 | AF213033 |
| 70 | 218381_s_at | U2AF2 | Hs.528007 | NM_007279 |
| 71 | 201947_s_at | CCT2 | Hs.189772 | NM_006431 |
| 72 | 212722_s_at | JMJD6 | Hs.514505 | AK021780 |
| 73 | 204825_at | MELK | Hs.184339 | NM_014791 |
| 74 | 203184_at | FBN2 | Hs.519294 | NM_001999 |
| 75 | 201266_at | TXNRD1 | Hs.708065 | NM_003330 |
| 76 | 202969_at | DYRK2 | Hs.173135 | AI216690 |
| 77 | 204817_at | ESPL1 | Hs.153479 | NM_012291 |
| 78 | 209523_at | TAF2 | Hs.122752 | AK001618 |
| 79 | 218491_s_at | THYN1 | Hs.13645 | NM_014174 |
| 80 | 217363_x_at | --- | --- | AL031313 |
| 81 | 218009_s_at | PRC1 | Hs.567385 | NM_003981 |
| 82 | 204026_s_at | ZWINT | Hs.591363 | NM_007057 |
| 83 | 218355_at | KIF4A | Hs.648326 | NM_012310 |
| 84 | 202153_s_at | NUP62 | Hs.574492 | NM_016553 |
| 85 | 213011_s_at | TPI1 | Hs.524219 | BF116254 |

(continued)

| Number | Probe set ID | Gene symbol | UniGene ID | GenBank accession number |
|--------|--------------|-------------|------------|--------------------------|
| 86 | 217966_s_at | FAM129A | Hs.518662 | NM_022083 |
| 87 | 214782_at | CTTN | Hs.596164 | AU155105 |
| 88 | 217967_s_at | FAM129A | Hs.518662 | AF288391 |
| 89 | 204649_at | TROAP | Hs.524399 | NM_005480 |
| 90 | 35671_at | GTF3C1 | Hs.371718 | U02619 |
| 91 | 213502_x_at | LOC91316 | Hs.148656 | AA398569 |
| 92 | 221285_at | ST8SIA2 | Hs.302341 | NM_006011 |
| 93 | 221519_at | FBXW4 | Hs.500822 | AF281859 |
| 94 | 202551_s_at | CRIM1 | Hs.699247 | BG546884 |
| 95 | 217138_x_at | IGL@ | Hs.449585 | AJ249377 |

When the subject is administered with anticancer agents as a preoperative adjuvant therapy, the method of the present embodiment may be combined with a publicly known method for predicting responsiveness to chemotherapy and prognosis. As such a method, a multigene assay, MPCP155 (see Tsunashima R. et al., Construction of multi-gene classifier for prediction of response to and prognosis after neoadjuvant chemotherapy for estrogen receptor positive breast cancers, Cancer Letters, 2015, vol. 365, p. 166-173) is known. In the present embodiment, the subject may be a subject whose responsiveness to chemotherapy has been determined, based on gene expression levels measured using probe sets specified by 155 probe set IDs shown in Table 4-1 and Table 4-5.

[Table 7]

Table 4-1

| Number | Probe set ID | Gene symbol |
|--------|--------------|-------------|
| 1 | 205896_at | SLC22A4 |
| 2 | 203685_at | BCL2 |
| 3 | 200970_s_at | SERP1 |
| 4 | 205355_at | ACADSB |
| 5 | 210678_s_at | AGPAT2 |
| 6 | 210046_s_at | IDH2 |
| 7 | 37152_at | PPARD |
| 8 | 205733_at | BLM |
| 9 | 215649_s_at | MVK |
| 10 | 214594_x_at | ATP8B1 |
| 11 | 218096_at | AGPAT5 |
| 12 | 203684_s_at | BCL2 |
| 13 | 202671_s_at | PDXK |
| 14 | 32837_at | AGPAT2 |
| 15 | 218923_at | CTBS |
| 16 | 200846_s_at | PPP1CA |
| 17 | 207005_s_at | BCL2 |

(continued)

| Number | Probe set ID | Gene symbol |
|--------|--------------|-------------|
| 18 | 221753_at | SSH1 |
| 19 | 208705_s_at | EIF5 |
| 20 | 207621_s_at | PEMT |
| 21 | 200849_s_at | AHCYL1 |
| 22 | 221065_s_at | CHST8 |
| 23 | 209281_s_at | ATP2B1 |
| 24 | 217006_x_at | FASN |
| 25 | 214095_at | SHMT2 |
| 26 | 213607_x_at | NADK |
| 27 | 206130_s_at | ASGR2 |
| 28 | 200784_s_at | LRP1 |
| 29 | 205301_s_at | OGG1 |
| 30 | 201627_s_at | INSIG1 |
| 31 | 205768_s_at | SLC27A2 |
| 32 | 203770_s_at | STS |
| 33 | 202376_at | SERPINA3 |
| 34 | 219723_x_at | AGPAT3 |
| 35 | 218506_x_at | GLYR1 |
| 36 | 208051_s_at | PAIP1 |
| 37 | 205769_at | SLC27A2 |

[Table 8]

Table 4-2

| Number | Probe set ID | Gene symbol |
|--------|--------------|-------------|
| 38 | 211762_s_at | KPNA2 |
| 39 | 204879_at | PDPN |
| 40 | 219429_at | FA2H |
| 41 | 211627_x_at | ESR1 |
| 42 | 202779_s_at | UBE2S |
| 43 | 210609_s_at | TP53I3 |
| 44 | 209064_x_at | PAIP1 |
| 45 | 208830_s_at | SUPT6H |
| 46 | 219048_at | PIGN |
| 47 | 209041_s_at | UBE2G2 |
| 48 | 201523_x_at | UBE2N |
| 49 | 208511_at | PTTG3P |

(continued)

| Number | Probe set ID | Gene symbol |
|--------|-------------|-------------|
| 50 | 222074_at | UROD |
| 51 | 209496_at | RARRES2 |
| 52 | 203366_at | POLG |
| 53 | 201782_s_at | AIP |
| 54 | 202715_at | CAD |
| 55 | 208624_s_at | EIF4G1 |
| 56 | 210449_x_at | MAPK14 |
| 57 | 201610_at | ICMT |
| 58 | 217294_s_at | ENO1 |
| 59 | 205140_at | FPGT |
| 60 | 204430_s_at | SLC2A5 |
| 61 | 204167_at | BTD |
| 62 | 203566_s_at | AGL |
| 63 | 210688_s_at | CPT1A |
| 64 | 213613_s_at | NADK |
| 65 | 215988_s_at | DLG1 |
| 66 | 206925_at | ST8SIA4 |
| 67 | 203365_s_at | MMP15 |
| 68 | 215842_s_at | ATP11A |
| 69 | 203939_at | NT5E |
| 70 | 201695_s_at | PNP |
| 71 | 200878_at | EPAS1 |
| 72 | 209740_s_at | PNPLA4 |
| 73 | 218313_s_at | GALNT7 |
| 74 | 209773_s_at | RRM2 |

[Table 9]

Table 4-3

| Number | Probe set ID | Gene symbol |
|--------|-------------|-------------|
| 75 | 217607_x_at | EIF4G2 |
| 76 | 205816_at | ITGB8 |
| 77 | 203244_at | PEX5 |
| 78 | 207851_s_at | INSR |
| 79 | 207275_s_at | ACSL1 |
| 80 | 204044_at | QPRT |
| 81 | 201218_at | CTBP2 |

(continued)

| Number | Probe set ID | Gene symbol |
|--------|--------------|-------------|
| 82 | 209916_at | DHTKD1 |
| 83 | 200785_s_at | LRP1 |
| 84 | 213343_s_at | GDPD5 |
| 85 | 218533_s_at | UCKL1 |
| 86 | 203381_s_at | APOE |
| 87 | 221142_s_at | PECR |
| 88 | 209533_s_at | PLAA |
| 89 | 218018_at | PDXK |
| 90 | 205225_at | ESR1 |
| 91 | 32502_at | GDPD5 |
| 92 | 201080_at | PIP4K2B |
| 93 | 208696_at | CCT5 |
| 94 | 209735_at | ABCG2 |
| 95 | 203554_x_at | PTTG1 |
| 96 | 206587_at | CCT6B |
| 97 | 202580_x_at | FOXM1 |
| 98 | 214210_at | SLC25A17 |
| 99 | 201291_s_at | TOP2A |
| 100 | 200675_at | CD81 |
| 101 | 208510_s_at | PPARG |
| 102 | 205128_x_at | PTGS1 |
| 103 | 216551_x_at | PLCG1 |
| 104 | 203634_s_at | CPT1A |
| 105 | 204946_s_at | TOP3A |
| 106 | 212883_at | APOE |
| 107 | 207076_s_at | ASS1 |
| 108 | 211026_s_at | MGLL |
| 109 | 200879_s_at | EPAS1 |
| 110 | 209537_at | EXTL2 |
| 111 | 221485_at | B4GALT5 |

[Table 10]

Table 4-4

| Number | Probe set ID | Gene symbol |
|--------|--------------|-------------|
| 112 | 220948_s_at | ATP1A1 |
| 113 | 218760_at | COQ6 |

(continued)

| Number | Probe set ID | Gene symbol |
| --- | --- | --- |
| 114 | 211233_x_at | ESR1 |
| 115 | 203343_at | UGDH |
| 116 | 217190_x_at | ESR1 |
| 117 | 203099_s_at | CDYL |
| 118 | 202533_s_at | DHFR |
| 119 | 206197_at | NME5 |
| 120 | 219718_at | FGGY |
| 121 | 201081_s_at | PIP4K2B |
| 122 | 215210_s_at | DLST |
| 123 | 211234_x_at | ESR1 |
| 124 | 203767_s_at | STS |
| 125 | 218924_s_at | CTBS |
| 126 | 204059_s_at | ME1 |
| 127 | 211754_s_at | SLC25A17 |
| 128 | 213279_at | DHRS1 |
| 129 | 217289_s_at | SLC37A4 |
| 130 | 204161_s_at | ENPP4 |
| 131 | 34187_at | RBMS2 |
| 132 | 202922_at | GCLC |
| 133 | 209616_s_at | CES1 |
| 134 | 218951_s_at | PLCXD1 |
| 135 | 40829_at | WDTC1 |
| 136 | 201117_s_at | CPE |
| 137 | 213379_at | COQ2 |
| 138 | 203633_at | CPT1A |
| 139 | 206311_s_at | PLA2G1B |
| 140 | 206630_at | TYR |
| 141 | 210745_at | ONECUT1 |
| 142 | 205552_s_at | OAS1 |
| 143 | 209425_at | AMACR |
| 144 | 201625_s_at | INSIG1 |
| 145 | 203072_at | MYOIE |
| 146 | 209998_at | PIGO |
| 147 | 201898_s_at | UBE2A |
| 148 | 204956_at | MTAP |

[Table 11]

Table 4-5

| Number | Probe set ID | Gene symbol |
|--------|--------------|-------------|
| 149 | 215966_x_at | GK3P |
| 150 | 209681_at | SLC19A2 |
| 151 | 59631_at | TXNRD3 |
| 152 | 219394_at | PGS1 |
| 153 | 206492_at | FHIT |
| 154 | 211738_x_at | CELA3A |
| 155 | 209165_at | AATF |

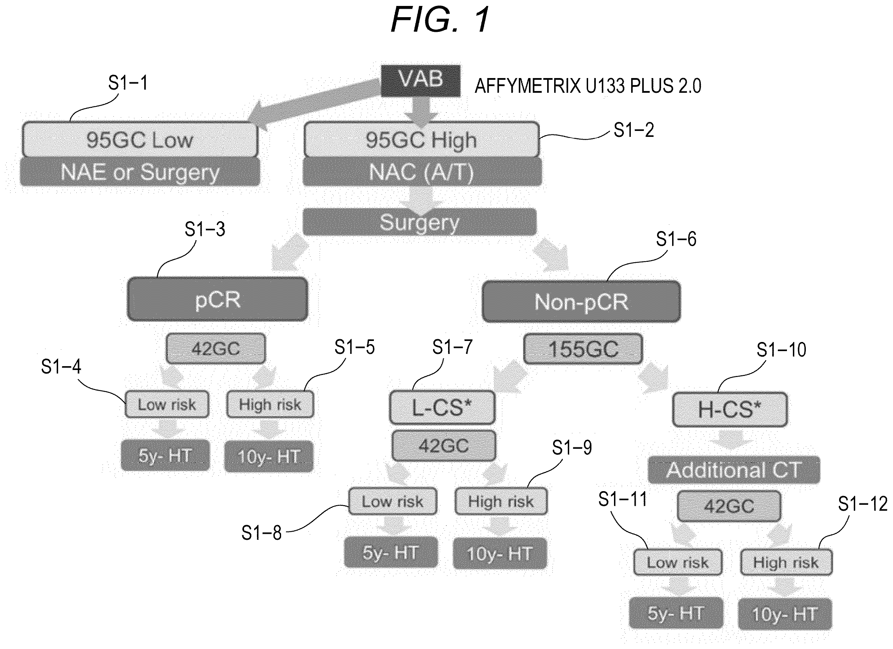

Flows when a treatment policy for breast cancer patients is decided by combining the method of the present embodiment with a publicly known multigene assay for predicting risk of early recurrence of breast cancer will be described with reference to Figs. 1 to 4. In any of Figs. 1 to 4, first, a biological sample is collected from a subject by vacuum-assisted breast biopsy (VAB). Then, tissue diagnosis is performed using a part of the biological sample to diagnose breast cancer. When diagnosed as breast cancer, RNA samples are prepared from the remaining biological sample, and expression levels of genes are measured using GeneChip (registered trademark) Human Genome U133 Plus 2.0 Array (Affymetrix, Inc.). This microarray contains not only the 42 probe sets used in the method of the present embodiment but also probe sets used in Curebest (registered trademark) 95GC Breast (hereinafter also referred to as "95GC") and MPCP155 (hereinafter also referred to as "155GC"). Accordingly, it is possible to acquire gene expression level data for a plurality of multigene assays by performing measurement by microarray only once, with respect to a biological sample obtained by biopsy for diagnosis of breast cancer. A multigene assay by microarray may further be combined with a multigene assay by RT-PCR method. The multigene assay by RT-PCR method includes Oncotype DX (registered trademark). In this case, first, tissue diagnosis is performed using a part of the biological sample obtained by VAB to diagnose breast cancer. When diagnosed as breast cancer, RNA samples are prepared from the remaining biological sample, and expression levels of genes are measured using GeneChip (registered trademark) Human Genome U133 Plus 2.0 Array (Affymetrix, Inc.) and RT-PCR method.

Referring to Fig. 1, first, a biological sample is collected from a subject by VAB, and diagnosis of breast cancer by tissue diagnosis and determination of risk of early recurrence by 95GC are performed. When it has been determined that the risk of early recurrence is low by 95GC, the process proceeds to step S1-1. In step S1-1, preoperative hormone therapy (NAE) or surgery is performed on the subject. When it has been determined that the risk of early recurrence is high by 95GC, the process proceeds to step S1-2. In step S1-2, preoperative chemotherapy (NAC (A/T)) with adriamycin or a taxane anticancer agent is performed on the subject, and then surgery is performed. When the postoperative course is pathologically complete remission (pCR), the process proceeds to step S1-3. In step S1-3, determination of risk of late recurrence by the method of the present embodiment (hereinafter also referred to as "42GC") is performed. When it has been determined that the risk of late recurrence is low by 42GC, the process proceeds to step S1-4. In step S1-4, hormone therapy for 5 years after surgery (5y-HT) is performed on the subject. When it has been determined that the risk of late recurrence is high by 42GC, the process proceeds to step S1-5. In step S1-5, hormone therapy for 10 years after surgery (10y-HT) is performed on the subject.

When the postoperative course in step S1-2 is pathologically complete non-remission (Non-pCR), the process proceeds to step S1-6. In step S1-6, determination of responsiveness to chemotherapy by 155GC is performed. When it has been determined that the responsiveness is low (L-CS*: low chemo-sensitivity) by 155GC, the process proceeds to step S1-7. In step S1-7, determination of the risk of late recurrence by 42GC is performed. When it has been determined that the risk of late recurrence is low by 42GC, the process proceeds to step S1-8, and when it has been determined that the risk of late recurrence is high, the process proceeds to step S1-9. Steps S1-8 and S1-9 are the same as those described for steps S1-4 and S1-5, respectively. In step S1-6, when it has been determined that the responsiveness is high (H-CS*: high chemo-sensitivity) by 155GC, the process proceeds to step S1-10. In step S1-10, additional chemotherapy (Additional CT) is performed on the subject and determination of the risk of late recurrence by 42GC is performed. When it has been determined that the risk of late recurrence is low by 42GC, the process proceeds to step S1-11, and when it has been determined that the risk of late recurrence is high, the process proceeds to step S1-12. Steps S1-11 and S1-12 are the same as those described for steps S1-4 and S1-5, respectively.

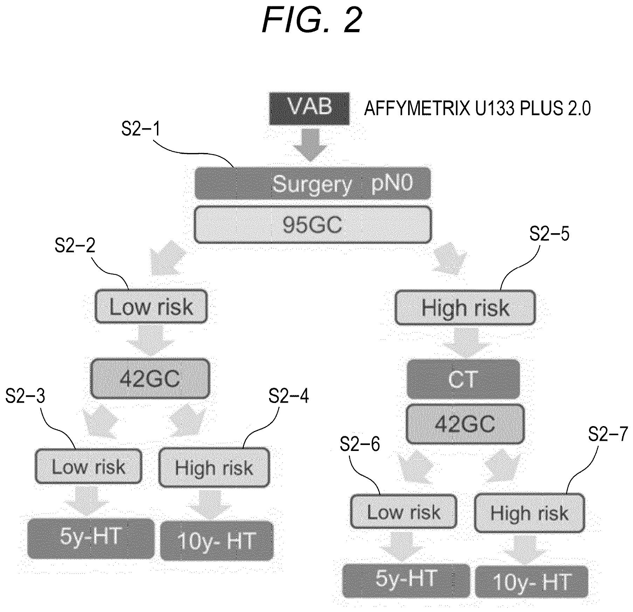

Referring to Fig. 2, first, a biological sample is collected from a subject by VAB, and diagnosis of breast cancer is performed by tissue diagnosis. When diagnosed as breast cancer, the process proceeds to step S2-1. In step S2-1, surgery is performed on the subject. When it has been determined that there is no lymph node metastasis (pN0) by postoperative pathological classification (pN classification), determination of the risk of early recurrence by 95GC is performed. When it has been determined that the risk of early recurrence is low by 95GC, the process proceeds to step S2-2. In step S2-2, determination of the risk of late recurrence by 42GC is performed on the subject. When it has been determined that the risk of late recurrence is low by 42GC, the process proceeds to step S2-3, and when it has been determined that the risk of late recurrence is high, the process proceeds to step S2-4. Steps S2-3 and S2-4 are the same as those described for steps S1-4 and S1-5, respectively. In step S2-1, when it has been determined that the risk of early recurrence is high by 95GC, the process proceeds to step S2-5. In step S2-5, chemotherapy (CT) is performed on the subject and determination of the risk of late recurrence by 42GC is performed. When it has been determined that the risk of late recurrence is low by 42GC, the process proceeds to step S2-6, and when it has been determined that the risk of late recurrence is high, the process proceeds to step S2-7. Steps S2-6 and S2-7 are the same as those described for steps S1-4 and S1-5, respectively.

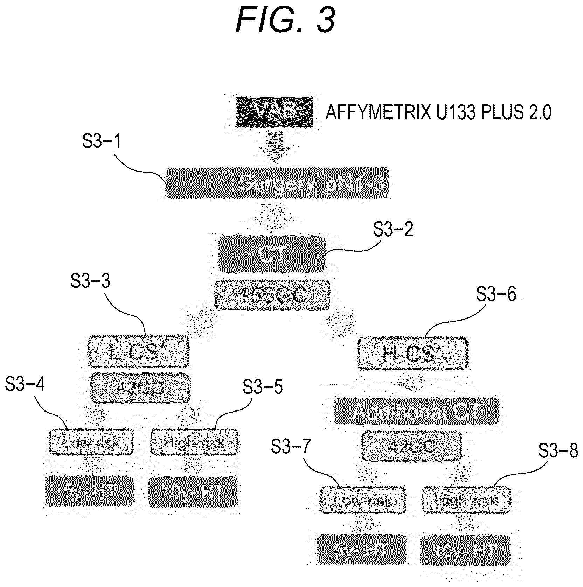

Referring to Fig. 3, first, a biological sample is collected from a subject by VAB, and diagnosis of breast cancer is performed by tissue diagnosis. When diagnosed as breast cancer, the process proceeds to step S3-1. In step S3-1, surgery is performed on the subject. When it has been determined that there is lymph node metastasis (pN1-3) by pN classification, the process proceeds to step S3-2. In step S3-2, chemotherapy (CT) is performed on the subject and determination of responsiveness to chemotherapy by 155GC is performed. When it has been determined that the responsiveness is low (L-CS*) by 155GC, the process proceeds to step S3-3. In step S3-3, determination of the risk of late recurrence by 42GC is performed. When it has been determined that the risk of late recurrence is low by 42GC, the process proceeds to step S3-4, and when it has been determined that the risk of late recurrence is high, the process proceeds to step S3-5. Steps S3-4 and S3-5 are the same as those described for steps S1-4 and S1-5, respectively In step S3-2, when it has been determined that the responsiveness is high (H-CS*) by 155GC, the process proceeds to step S3-6. In step S3-6, additional chemotherapy (Additional CT) is performed on the subject and determination of the risk of late recurrence by 42GC is performed. When it has been determined that the risk of late recurrence is low by 42GC, the process proceeds to step S3-7, and when it has been determined that the risk of late recurrence is high, the process proceeds to step S3-8. Steps S3-7 and S3-8 are the same as those described for steps S1-4 and S1-5, respectively

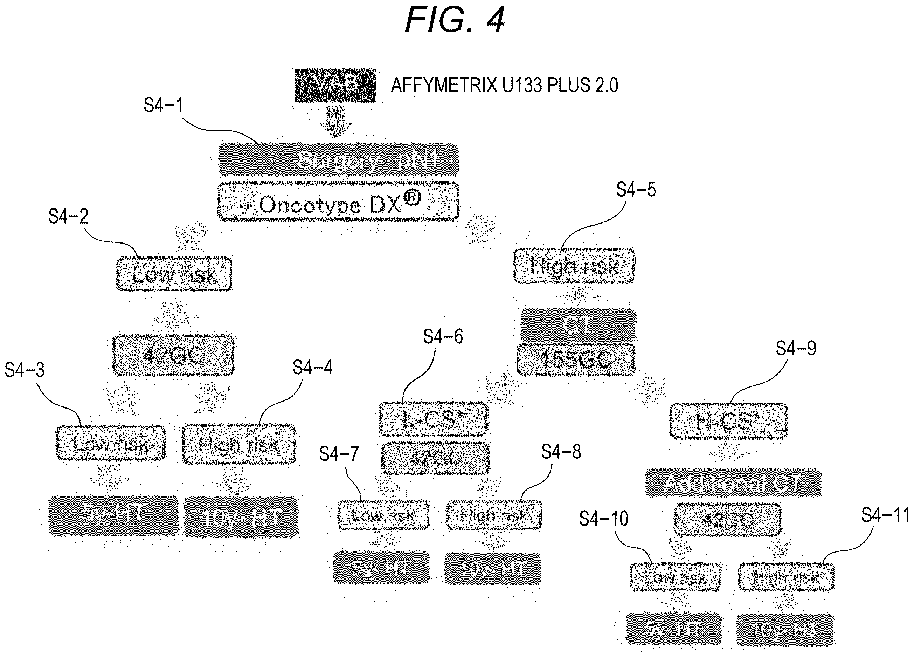

Referring to Fig. 4, first, a biological sample is collected from a subject by VAB, and diagnosis of breast cancer is performed by tissue diagnosis. When diagnosed as breast cancer, the process proceeds to step S4-1. In step S4-1, surgery is performed on the subject. When it has been determined that there is lymph node metastasis (pN1) by pN classification, determination of the risk of early recurrence by Oncotype DX (registered trademark) is performed. When it has been determined that the risk of early recurrence is low by Oncotype DX (registered trademark), the process proceeds to step S4-2. In step S4-2, determination of the risk of late recurrence by 42GC is performed on the subject. When it has been determined that the risk of late recurrence is low by 42GC, the process proceeds to step S4-3, and when it has been determined that the risk of late recurrence is high, the process proceeds to step S4-4. Steps S4-3 and S4-4 are the same as those described for steps S1-4 and S1-5, respectively In step S4-1, when it has been determined that the risk of early recurrence is high by Oncotype DX (registered trademark), the process proceeds to step S4-5. In step S4-5, chemotherapy (CT) is performed on the subject and determination of responsiveness to chemotherapy by 155GC is performed. When it has been determined that the responsiveness is low (L-CS*) by 155GC, the process proceeds to step S4-6. In step S4-6, determination of the risk of late recurrence by 42GC is performed. When it has been determined that the risk of late recurrence is low by 42GC, the process proceeds to step S4-7, and when it has been determined that the risk of late recurrence is high, the process proceeds to step S4-8. Steps S4-7 and S4-8 are the same as those described for steps S1-4 and S1-5, respectively In step S4-5, when it has been determined that the responsiveness is high (H-CS*) by 155GC, the process proceeds to step S4-9. In step S4-9, additional chemotherapy (Additional CT) is performed on the subject and determination of the risk of late recurrence by 42GC is performed. When it has been determined that the risk of late recurrence is low by 42GC, the process proceeds to step S4-10, and when it has been determined that the risk of late recurrence is high, the process proceeds to step S4-11. Steps S4-10 and S4-11 are the same as those described for steps S1-4 and S1-5, respectively

[2. Determination Device and Computer Program]

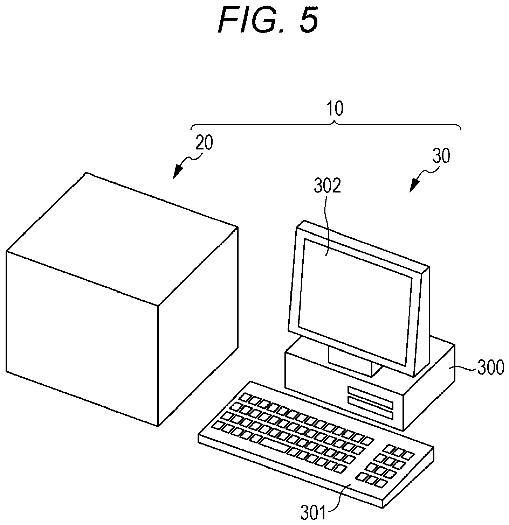

The scope of the present disclosure also includes a device and a computer program for implementing the above-described method of the present embodiment. An example of the device for determining prognosis of breast cancer of the present embodiment will be described with reference to the drawings. However, the present embodiment is not limited only to the embodiment shown in this example. A determination device 10 shown in Fig. 5 includes a measuring device 20 and a computer system 30 connected to the measuring device 20.

In this example, the measuring device 20 is a microarray scanner that detects a signal based on a target nucleic acid hybridized to a probe on a microarray. The signal is optical information such as a fluorescence signal. In this case, when the microarray that is brought into contact with the RNA sample is set in the measuring device 20, the measuring device 20 acquires optical information based on the target nucleic acid hybridized to the probe on the microarray, and the measuring device 20 transmits the acquired optical information to the computer system 30.

The microarray scanner is not particularly limited as long as it can detect a signal based on the target nucleic acid hybridized to the probe. Since the type of the signal differs depending on the labeling substance used for labeling the target nucleic acid, the microarray scanner can be appropriately selected according to the type of the labeling substance. For example, when the labeling substance is a fluorescent substance, a microarray scanner capable of detecting a fluorescence signal can be used as the measuring device 20.

When expression levels of genes are analyzed by a nucleic acid amplification method such as quantitative RT-PCR, the measuring device 20 may be a nucleic acid amplification detection apparatus. In this case, a reaction solution containing an RNA sample, an enzyme for nucleic acid amplification, a primer and the like is set in the measuring device 20, and a nucleic acid in the reaction solution is amplified by the nucleic acid amplification method. The measuring device 20 acquires optical information such as fluorescence generated from the reaction solution and turbidity of the reaction solution by an amplification reaction, and the measuring device 20 transmits the optical information to the computer system 30.

The computer system 30 includes a computer main body 300, an input unit 301, and a display unit 302 that displays specimen information, a determination result, and the like. The computer system 30 receives the optical information from the measuring device 20. Then, the processor of the computer system 30 executes a program for determining prognosis of breast cancer, based on the optical information. As shown in Fig. 5, the computer system 30 may be equipment separate from the measuring device 20, or may be equipment including the measuring device 20. In the latter case, the computer system 30 may itself be the determination device 10.

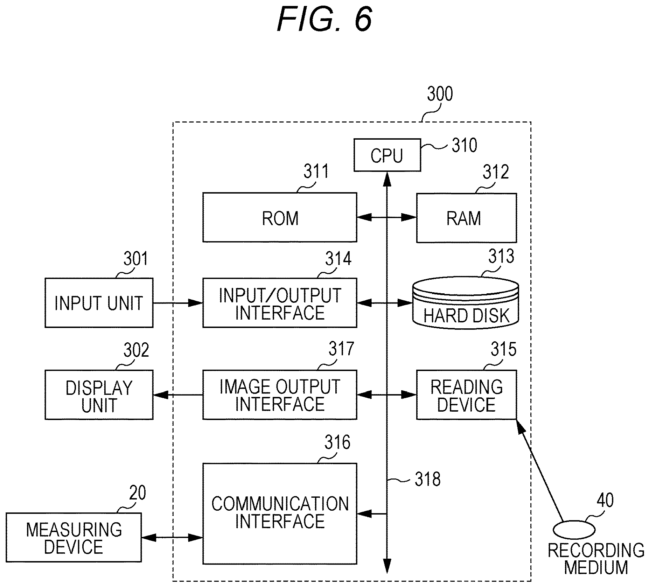

Referring to Fig. 6, the computer main body 300 includes a central processing unit (CPU) 310, a read only memory (ROM) 311, a random access memory (RAM) 312, a hard disk 313, an input/output interface 314, a reading device 315, a communication interface 316, and an image output interface 317. The CPU 310, the ROM 311, the RAM 312, the hard disk 313, the input/output interface 314, the reading device 315, the communication interface 316 and the image output interface 317 are data-communicably connected by a bus 318. The measuring device 20 is communicably connected to the computer system 30 via the communication interface 316.

The CPU 310 can execute a program stored in the ROM 311 or the hard disk 313 and a program loaded in the RAM 312. The CPU 310 calculates fluorescence intensity based on the optical information acquired from the measuring device 20. The CPU 310 calculates solution D according to a discriminant represented by formula (I) stored in the ROM 311 or the hard disk 313. Then, the CPU 310 determines prognosis of breast cancer based on the acquired solution D and the determination criteria stored in the ROM 311 or the hard disk 313. The CPU 310 outputs the determination result and displays the determination result on the display unit 302.

The ROM 311 includes a mask ROM, PROM, EPROM, EEPROM, and the like. In the ROM 311, a computer program executed by the CPU 310 and data used for executing the computer program are recorded.

The RAM 312 includes SRAM, DRAM, and the like. The RAM 312 is used for reading the program recorded in the ROM 311 and the hard disk 313. The RAM 312 is also used as a work area of the CPU 310 when these programs are executed.

The hard disk 313 has installed therein an operating system to be executed by the CPU 310, a computer program such as an application program (the program for determining prognosis of breast cancer), and data used for executing the computer program.

The reading device 315 includes a flexible disk drive, a CD-ROM drive, a DVD-ROM drive, and the like. The reading device 315 can read a program or data recorded on a portable recording medium 40.

The input/output interface 314 includes, for example, a serial interface such as USB, IEEE1394 and RS-232C, a parallel interface such as SCSI, IDE and IEEE1284, and an analog interface including a D/A converter, an A/D converter and the like. The input unit 301 such as a keyboard and a mouse is connected to the input/output interface 314. An operator can input various commands to the computer main body 300 through the input unit 301.

The communication interface 316 is, for example, an Ethernet (registered trademark) interface or the like. The computer main body 300 can also transmit print data to a printer or the like through the communication interface 316.

The image output interface 317 is connected to the display unit 302 including an LCD, a CRT, and the like. As a result, the display unit 302 can output a video signal corresponding to the image data coming from the CPU 310. The display unit 302 displays an image (screen) according to the input video signal.

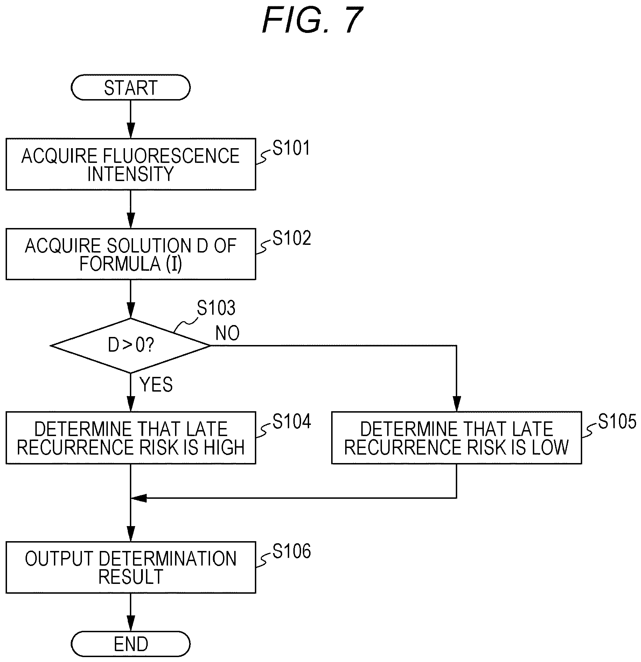

Referring to Fig. 7, a processing procedure for determining prognosis of breast cancer executed by the determination device 10 will be described. Here, a case where determination of the risk of late recurrence is performed based on a fluorescence signal generated from the target nucleic acid bound to the probe on the microarray will be described as an example. However, the present embodiment is not limited to this example.

In step S101, the CPU 310 acquires optical information (fluorescence signal) from the measuring device 20, the CPU 310 calculates a fluorescence intensity from the acquired optical information, and the CPU 310 stores the calculated fluorescence intensity in the hard disk 313. In step S102, the CPU 310 calculates solution D according to the discriminant represented by the formula (I) stored in the hard disk 313, using the calculated fluorescence intensity, and the CPU 310 stores the solution D in the hard disk 313. In step S103, the CPU 310 compares the calculated solution D with the determination criteria stored in the hard disk 313. When the solution D is a positive value, the process proceeds to step S104, and a determination result indicating that the late recurrence risk of the subject is high is stored in the hard disk 313. When the solution D is zero or a negative value, the process proceeds to step S105, and a determination result indicating that the late recurrence risk of the subject is low is stored in the hard disk 313. In step S105, the CPU 310 outputs the determination result, and the CPU 310 displays the determination result on the display unit 302, or the CPU 310 makes a printer print out the determination result. Accordingly, it is possible to provide doctors and the like with information to assist the determination of prognosis of breast cancer.

Hereinafter, the present disclosure will be described in more detail by examples, but the present disclosure is not limited to these examples.

EXAMPLES

Example 1: Search for Predictors of Late Recurrence

(1) Acquisition of gene expression levels

Data of breast cancer patients (779 cases) shown in Table 5 were extracted from four data sets of accession numbers: GSE6532, GSE12093, GSE17705 and GSE26971 in NCBI Gene Expression Omnibus (http://www.ncbi.nlm.nih.gov/geo/) of microarray experiments. All of these patients were ER positive and also received only administration of hormones as a treatment. In the table, "cT" is a clinical tumor diameter, "cN" is a clinical nodal state (presence or absence of lymph node metastasis), and "PR" is a progesterone receptor. The term "95GC" indicates risk of early recurrence determined by Curebest (registered trademark) 95GC Breast (Sysmex Corporation). The term "155GC" indicates responsiveness to chemotherapy (chemo-sensitivity) determined by MPCP155 (see Non-Patent Document 1). Hereinbelow, among patients with recurrence (177 cases), patients with recurrence within 5 years ($\leq$ 5 years) after surgery are called "early recurrence group", and patients with recurrence more than 5 years (> 5 years) after surgery are called "late recurrence group".

[Table 12]

| Table 5 | | | | | |
|---|---|---|---|---|---|
| | Training set | | | | |
| | GSE6532 | GSE12093 | GSE17705 | GSE26971 | Total |
| Number of cases | 87 | 136 | 298 | 258 | 779 |
| Age | | | | | |
| < 50 | 3 | NA | NA | NA | 3 |
| $\geq$ 50 | 84 | NA | NA | NA | 84 |
| cT | | | | | |
| T1 | 43 | NA | NA | 102 | 145 |
| T2/3/4 | 44 | NA | NA | 145 | 189 |
| Unknown | 0 | NA | NA | 11 | 11 |
| cN | | | | | |
| Positive | 58 | 0 | 112 | 87 | 257 |
| Negative | 29 | 136 | 175 | 129 | 469 |
| Unknown | 0 | 0 | 11 | 42 | 53 |
| Histological grade | | | | | |
| 1 | 17 | NA | NA | NA | 17 |
| 2/3 | 53 | NA | NA | NA | 53 |
| Unknown | 17 | NA | NA | NA | 17 |

(continued)

| | | | | | |
|---|---|---|---|---|---|
| PR | | | | | |
| Positive | 64 | NA | NA | NA | 64 |
| Negative | 21 | NA | NA | NA | 21 |
| Unknown | 2 | NA | NA | NA | 2 |
| Recurrence | 28 | 20 | 71 | 58 | 177 |
| $\leq$ 5 yrs | 14 | 12 | 42 | 41 | 109 |
| > 5 yrs | 14 | 8 | 29 | 17 | 68 |
| No recurrence | 59 | 116 | 227 | 200 | 602 |
| Sub type | | | | | |
| Luminal A | 37 | 65 | 148 | 132 | 382 |
| Luminal B | 50 | 71 | 150 | 126 | 397 |
| 95GC | | | | | |
| Low risk | 48 | 85 | 177 | 151 | 461 |
| High risk | 39 | 51 | 121 | 107 | 318 |
| 155GC | | | | | |
| Low sensitivity | 42 | 61 | 146 | 113 | 362 |
| High sensitivity | 45 | 75 | 152 | 145 | 417 |

(2) Analysis of gene expression level data

Gene expression level data (fluorescence intensity data) of the breast cancer patients was normalized by using CEL file data of each data set and MAS5 statistical algorithm of analysis software (Affymetrix Expression Console (trademark) software, manufactured by Affymetrix, Inc.). Next, for each data set, from a value of a gene expression level measured by each probe set of the microarray, a mean value of the gene expression levels in the data set was subtracted to standardize the value of the expression level of each gene (mean-centering). Then, z-score was calculated for each probe set on the microarray, by using a package "GeneMeta v1 .16.0" (http://www.bioconductor.org/packages/2.4/bioc/html/GeneMeta.html) contained in an additional package "BioConductor" ver. 2.4 used in software for statistical analysis "R", according to a literature by Choi J.K et al. (Combining multiple microarray studies and modeling interstudy variation, Bioinformatics, 2003, vol. 19, suppl. 1, p. i84-94).

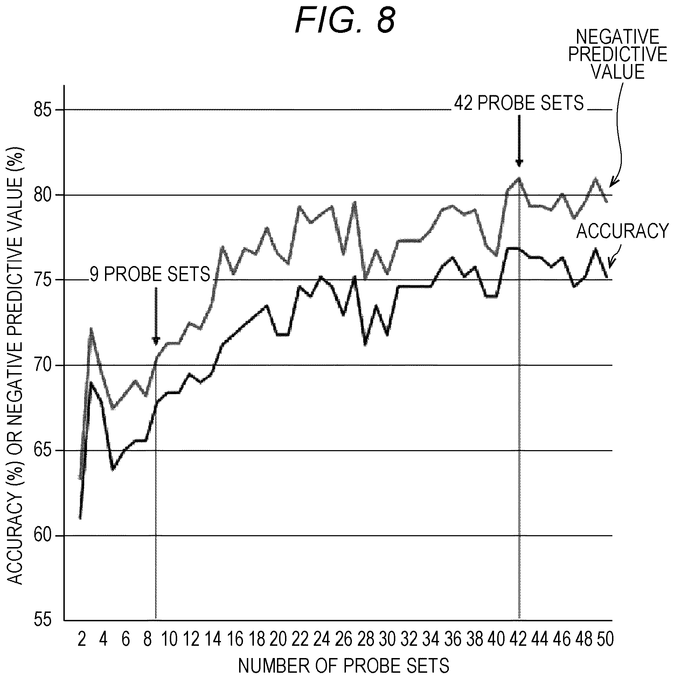

It was evaluated by chi-square test that the expression level of the gene corresponding to the probe set was different between the early recurrence group and the late recurrence group, and a probe set with p value of less than 0.01 was selected. Next, using a diagonal linear discriminant analysis (DLDA) as an algorithm of a discriminant, a probe set group was selected while increasing the number of the probe sets to be selected one by one in ascending order of the p value in the chi-square test, and a discriminant was constructed. Using the obtained discriminant and data of the above 177 recurrent patients, the number of probe sets with which accuracy and negative predictive value are maximized were determined by Leave-One-Out Cross-Validation method. The accuracy refers to a ratio obtained by dividing the sum of "the number of cases in which late recurrence was predicted, and late recurrence occurred" and "the number of cases in which late recurrence was not predicted, and late recurrence did not occur" by "the total number of cases". The negative predictive value is a ratio obtained by dividing "the number of cases in which late recurrence was not predicted, and late recurrence did not occur" by "the number of cases in which late recurrence was not predicted". The results are shown in Fig. 8.

(3) Results

From Fig. 8, it was found that the negative predictive value and the accuracy were maximized when the number of probe sets was 42. It was found that the negative predictive value was at least 70% when the number of probe sets was nine or more. As described above, the negative predictive value is a probability that late recurrence actually does not occur when it is predicted that late recurrence does not occur. The selected 42 probe sets correspond to genes whose expression levels were significantly different between the late recurrence group and the early recurrence group. Therefore, when it is predicted that late recurrence does not occur based on the expression levels of genes measured by the probe sets, it means the same as the case where it is predicted that early recurrence occurs. That is, the negative predictive value can be rephrased as a probability that early recurrence actually occurs when it is predicted that early recurrence occurs.

IDs, z-scores, p values of chi-square test and weighting factors in the discriminants of the selected 42 probe sets are shown in Table 6-1 and Table 6-2. In which of the late recurrence group and the early recurrence group each gene is highly expressed is also shown in Table 6-1 and Table 6-2. In the table, "late" refers to a late recurrence group, "early" refers to an early recurrence group. The probe set IDs are IDs assigned to the probe sets of GeneChip (registered trademark) Human Genome U133 Plus 2.0 Array (Affymetrix, Inc.).

[Table 13]

Table 6-1

| Gene number | Probe set ID | Gene symbol | Z-score | P value | Weighting factor | High expression |
|---|---|---|---|---|---|---|
| 1 | 212196_at | IL6ST | -4.12792 | 0.0000366 | 1.43235997 | Late |
| 2 | 205440_s_at | NPY1R | -3.88231 | 0.000103 | 0.450736337 | Late |
| 3 | 208788_at | ELOVL5 | -3.72932 | 0.000192 | 1.793041948 | Late |
| 4 | 214306_at | OPA1 | 3.70520 | 0.000211 | -2.545950461 | Early |
| 5 | 212195_at | IL6ST | -3.66917 | 0.000243 | 1.539087017 | Late |
| 6 | 210980_s_at | ASAH1 | -3.65969 | 0.000252 | 1.539921375 | Late |
| 7 | 204864_s_at | IL6ST | -3.65831 | 0.000253 | 1.151743979 | Late |
| 8 | 221590_s_at | ALDH6A1 | -3.59018 | 0.000330 | 1.837908119 | Late |
| 9 | 218692_at | SYBU | -3.57397 | 0.000351 | 1.149172541 | Late |
| 10 | 205428_s_at | CALB2 | 3.55933 | 0.000371 | -1.179415269 | Early |
| 11 | 206414_s_at | ASAP2 | 3.55143 | 0.000383 | -2.323532179 | Early |
| 12 | 201156_s_at | RAB5C | -3.54402 | 0.000394 | 2.316847761 | Late |
| 13 | 216988_s_at | PTP4A2 | -3.52418 | 0.000424 | 2.002072892 | Late |
| 14 | 213485_s_at | ABCC10 | 3.51999 | 0.000431 | -2.306701292 | Early |
| 15 | 206825_at | OXTR | 3.51415 | 0.000441 | -1.1298499 | Early |
| 16 | 211538_s_at | HSPA2 | -3.50756 | 0.000452 | 0.964294299 | Late |
| 17 | 205596_s_at | SMURF2 | 3.49837 | 0.000468 | -2.109236485 | Early |
| 18 | 202752_x_at | SLC7A8 | -3.46945 | 0.000521 | 1.363057605 | Late |
| 19 | 214435_x_at | RALA | 3.45260 | 0.000555 | -2.288374092 | Early |
| 20 | 209869_at | ADRA2A | -3.44181 | 0.000577 | 1.377444437 | Late |
| 21 | 203360_s_at | MYCBP | -3.39767 | 0.000679 | 1.688897659 | Late |
| 22 | 205898_at | CX3CR1 | -3.39344 | 0.000690 | 0.740592577 | Late |
| 23 | 203720_s_at | ERCC1 | -3.37674 | 0.000733 | 1.658860215 | Late |
| 24 | 205963_s_at | DNAJA3 | -3.36349 | 0.000769 | 2.534304027 | Late |
| 25 | 211000_s_at | IL6ST | -3.31557 | 0.000914 | 0.971312607 | Late |
| 26 | 203045_at | NINJ1 | -3.29288 | 0.000991 | 1.889380552 | Late |
| 27 | 218513_at | C4orf43 | -3.26230 | 0.001105 | 1.429869188 | Late |
| 28 | 209417_s_at | TFT35 | -3.26153 | 0.001108 | 1.323441517 | Late |
| 29 | 213527_s_at | ZNF688 | -3.25889 | 0.001118 | 2.158913805 | Late |
| 30 | 201716_at | SNX1 | -3.25195 | 0.001146 | 2.2492475 | Late |

(continued)

| Gene number | Probe set ID | Gene symbol | Z-score | P value | Weighting factor | High expression |
|---|---|---|---|---|---|---|
| 31 | 201988_s_at | CREBL2 | -3.22177 | 0.001274 | 1.921069642 | Late |
| 32 | 204934_s_at | HPN | -3.21415 | 0.001308 | 1.115597062 | Late |
| 33 | 204862_s_at | NME3 | -3.13797 | 0.001701 | 1.661682832 | Late |
| 34 | 203769_s_at | STS | 3.12877 | 0.001755 | -1.392023997 | Early |
| 35 | 204334_at | KLF7 | 3.10363 | 0.001911 | -1.662199312 | Early |
| 36 | 208911_s_at | PDHB | -3.09973 | 0.001936 | 2.071027409 | Late |
| 37 | 204863_s_at | IL6ST | -3.09878 | 0.001943 | 0.825924727 | Late |

[Table 14]

Table 6-2

| Gene number | Probe set ID | Gene symbol | Z-score | P value | Weighting factor | High expression |
|---|---|---|---|---|---|---|
| 38 | 209706_at | NKX3-1 | -3.08717 | 0.002020 | 0.611126615 | Late |
| 39 | 203733_at | DEXI | -3.07032 | 0.002138 | 1.847912036 | Late |
| 40 | 213702_x_at | ASAH1 | -3.06744 | 0.002158 | 1.575733773 | Late |
| 41 | 202554_s_at | GSTM3 | -3.05803 | 0.002227 | 0.648154701 | Late |
| 42 | 221515_s_at | LCMT1 | -3.04479 | 0.002328 | 1.7259299 | Late |

Based on the above results, a final discriminant was constructed. The obtained discriminant is represented by the following formula (I).

$$D = \Sigma_i(w_i \times y_i) - (-4.763756453) \cdots(I)$$

wherein i represents a number assigned to each gene shown in Table 6-1 and Table 6-2, $w_i$ represents a weighting factor of gene numbered i shown in Table 6-1 and Table 6-2, $y_i$ represents a standardized expression level of gene according to a formula represented by formula (II):

$$y_i = x_i - m_i \cdots(II)$$

wherein $x_i$ represents an expression level of gene numbered i shown in Table 6-1 and Table 6-2, and $m_i$ represents a mean value of expression levels of genes numbered i shown in Table 6-1 and Table 6-2 over specimens, and $\Sigma_i$ represents a sum total over the genes.

When solution D of the discriminant is a positive value, it is predicted that risk of late recurrence of the subject is high. When the solution D is zero or a negative value, it is predicted that the risk of late recurrence of the subject is low. When it is predicted that the risk of late recurrence is high, there is a possibility that micrometastasis of breast cancer with slow growth rate may exist in the body of the subject even if no recurrence is observed for 5 years after surgery, and extended endocrine treatment can be applied to the subject. As described above, the prediction that the risk of late recurrence is low can be rephrased as a prediction that the risk of early recurrence is high. When breast cancer does not recur for 5 years after surgery in a subject predicted to have a low risk of late recurrence, it can be considered that breast cancer has been cured.

Example 2: Verification of predictors of late recurrence

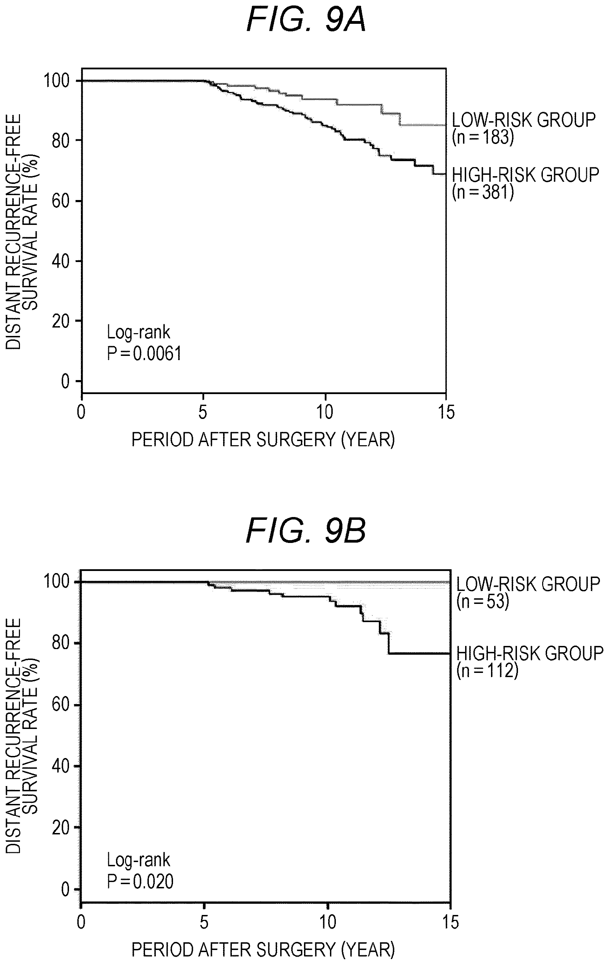

Among a training set of Example 1, the risk of late recurrence of breast cancer was predicted for ER-positive breast cancer patients (564 cases) who did not recur for 5 years after surgery, by the discriminant represented by the formula (I). For patients (381 cases) predicted to have a high risk of late recurrence and patients (183 cases) predicted to have a low risk of late recurrence, the distant recurrence-free survival rate (DRFS rate) for 15 years after surgery was examined by Kaplan-Meier plot. Significant differences were evaluated by log-rank test. The results are shown in Fig. 9A.

Gene expression level data of ER-positive breast cancer patients (165 cases) who did not recur for 5 years after surgery, which are different from the breast cancer patients of the training set, was used as a validation set. Using the data, the risk of late recurrence was predicted in the same manner as above. For patients (112 cases) predicted to have a high risk of late recurrence and patients (53 cases) predicted to have a low risk of late recurrence, the distant recurrence-free survival rate for 15 years after surgery was examined by Kaplan-Meier plot. Significant differences were evaluated by log-rank test. The results are shown in Fig. 9B.

From Fig. 9A, the distant recurrence-free survival rate for 15 years after surgery was about 70% for patients predicted to have a high risk of late recurrence ("high-risk group" in the figure), whereas it was about 85% for patients predicted to have a low risk of late recurrence ("low-risk group" in the figure). p was 0.0061 by log-rank test. Likewise, from Fig. 9B, the distant recurrence-free survival rate for 15 years after surgery was about 75% in the high-risk group, whereas it was 100% in the low-risk group. p was 0.020 by log-rank test. Accordingly, it can be said that the risk of late recurrence is significantly lower in the low-risk group than in the high-risk group. From these results, it was shown that the risk of late recurrence of breast cancer can be predicted with high accuracy by analyzing the gene expression levels measured using the probes specified by the probe set IDs shown in Table 6-1 and Table 6-2.

**Claims**

1. A method for acquiring information on prognosis of estrogen receptor (ER)-positive breast cancer, the method comprising:

    a measuring step of measuring expression levels of a first gene group, said gene group consisting of IL6ST, NPY1R, ELOVL5, OPA1, ASAH1, ALDH6A1 and SYBU, in an RNA sample prepared from a biological sample collected from a subject;
    an analyzing step of analyzing the measured expression levels of genes; and
    an acquiring step of acquiring information on prognosis of ER-positive breast cancer based on the sum of weighted standardized expression levels of said gene group, wherein each said standardized expression level is the measured gene expression level corrected by a mean gene expression level of RNA samples derived from a plurality of subjects.

2. The method of claim 1, wherein said plurality of subjects are ER positive breast cancer patients.

3. The method according to claims 1 or 2, wherein, in the measuring step, the expression levels are measured using a microarray.

4. The method according to claims 1 or 2, wherein, in the measuring step, an expression level of at least one gene selected from CALB2, ASAP2, RAB5C, PTP4A2, ABCC10, OXTR, HSPA2, SMURF2, SLC7A8, RALA, ADRA2A, MYCBP, CX3CR1, ERCC1, DNAJA3, NINJ1, C4orf43, IFI35, ZNF688, SNX1, CREBL2, HPN, NME3, STS, KLF7, PDHB, NKX3-1, DEXI, GSTM3 and LCMT1 is further measured.

5. The method according to claim 4, wherein, in the measuring step, the expression levels of said genes is measured using a microarray.

6. The method according to any one of claims 1 to 5, wherein the information on prognosis of breast cancer is information on risk of recurrence after surgery.

7. The method according to claim 6, wherein the information on risk of recurrence after surgery is information on risk of late recurrence after surgery.

8. The method according to claim 7, wherein the information on risk of late recurrence is information on risk of recurrence more than 5 years within 10 years after surgery.

9. The method according to any one of claims 1 to 8, wherein, in the analyzing step, the expression level is analyzed using a classification method, a hierarchical cluster analysis or a scoring method.

10. The method according to claim 9, wherein the classification method is a diagonal linear discriminant analysis or between-group analysis.

11. The method according to any one of claims 1 to 9, wherein, in the analyzing step, using the expression level and a discriminant represented by the following formula (1), solution D of the discriminant is determined, and
in the acquiring step, risk of late recurrence of the subject is determined to be high when the solution D is a positive value, and risk of late recurrence of the subject is determined to be low when the solution D is zero or a negative value.

$$D = \Sigma_i(w_i \times y_i) - (-4.763756453) \cdots (I)$$

wherein i represents a number assigned to each gene shown in Table 1-1 and Table 1-2, $w_i$ represents a weighting factor of gene numbered i shown in Table 1-1 and Table 1-2, $y_i$ represents a standardized expression level of gene according to a formula represented by formula (II):

$$y_i = x_i - m_i \cdots (II)$$

wherein $x_i$ represents an expression level of gene numbered i shown in Table 1-1 and Table 1-2, and $m_i$ represents a mean value of expression levels of genes numbered i shown in Table 1-1 and Table 1-2 over specimens, and $\Sigma_i$ represents a sum total over the genes.

Table 1-1

| Gene number | Gene symbol | Weighting factor |
|---|---|---|
| 1 | IL6ST | 1.43235997 |
| 2 | NPY1R | 0.450736337 |
| 3 | ELOVL5 | 1.793041948 |
| 4 | OPA1 | -2.545950461 |
| 5 | IL6ST | 1.539087017 |
| 6 | ASAH1 | 1.539921375 |
| 7 | IL6ST | 1.151743979 |
| 8 | ALDH6A1 | 1.837908119 |
| 9 | SYBU | 1.149172541 |

Table 1-2

| Gene number | Gene symbol | Weighting factor |
|---|---|---|
| 10 | CALB2 | -1.179415269 |
| 11 | ASAP2 | -2.323532179 |
| 12 | RAB5C | 2.316847761 |
| 13 | PTP4A2 | 2.002072892 |
| 14 | ABCC10 | -2.306701292 |
| 15 | OXTR | -1.1298499 |
| 16 | HSPA2 | 0.964294299 |
| 17 | SMURF2 | -2.109236485 |

(continued)

| Gene number | Gene symbol | Weighting factor |
|---|---|---|
| 18 | SLC7A8 | 1.363057605 |
| 19 | RALA | -2.288374092 |
| 20 | ADRA2A | 1.377444437 |
| 21 | MYCBP | 1.688897659 |
| 22 | CX3CR1 | 0.740592577 |
| 23 | ERCC1 | 1.658860215 |
| 24 | DNAJA3 | 2.534304027 |
| 25 | IL6ST | 0.971312607 |
| 26 | NINJ1 | 1.889380552 |
| 27 | C4orf43 | 1.429869188 |
| 28 | IFI35 | 1.323441517 |
| 29 | ZNF688 | 2.158913805 |
| 30 | SNX1 | 2.2492475 |
| 31 | CREBL2 | 1.921069642 |
| 32 | HPN | 1.115597062 |
| 33 | NME3 | 1.661682832 |
| 34 | STS | -1.392023997 |
| 35 | KLF7 | -1.662199312 |
| 36 | PDHB | 2.071027409 |
| 37 | IL6ST | 0.825924727 |
| 38 | NKX3-1 | 0.611126615 |
| 39 | DEXI | 1.847912036 |
| 40 | ASAH1 | 1.575733773 |
| 41 | GSTM3 | 0.648154701 |
| 42 | LCMT1 | 1.7259299 |

12. The method according to any one of claims 1 to 11, wherein the subject is a subject who did not have recurrence of breast cancer for 5 years after surgery.

13. The method according to any one of claims 1 to 12, wherein the subject is a subject whose risk of early recurrence of breast cancer has been determined to be low.

14. The method according to claim 13, wherein the subject is a subject whose risk of early recurrence of breast cancer has been determined to be low, based on gene expression levels measured using a microarray comprising probe sets specific for the 95 genes shown in Table 2-1 and Table 2-2.

Table 2-1

| Number | Gene symbol | UniGene ID | GenBank accession number |
|---|---|---|---|
| 1 | KIF15 | Hs.646856 | NM 020242 |
| 2 | DTL | Hs.656473 | NM 016448 |
| 3 | DONSON | Hs.436341 | AF232674 |

(continued)

| Number | Gene symbol | UniGene ID | GenBank accession number |
|---|---|---|---|
| 4 | KPNA2 | Hs.594238 | NM 002266 |
| 5 | PNRC1 | Hs.75969 | AF279899 |
| 6 | MED14 | Hs.407604 | AF135802 |
| 7 | KLC2 | Hs.280792 | NM 022822 |
| 8 | JMJD6 | Hs.514505 | AK021780 |
| 9 | LRRC59 | Hs.370927 | AK025328 |
| 10 | CAND1 | Hs.546407 | AB020636 |
| 11 | NUSAP1 | Hs.615092 | NM_016359 |
| 12 | CCBL2 | Hs.481898 | BC000819 |
| 13 | KIAA0406 | Hs.655481 | AB007866 |
| 14 | PLOD2 | Hs.477866 | NM_000935 |
| 15 | CTTN | Hs.596164 | NM_005231 |
| 16 | HSPB1 | Hs.520973 | NM_001540 |
| 17 | BUB1B | Hs.631699 | NM_001211 |
| 18 | TUBA1C | Hs.719091 | BC005946 |
| 19 | ESPL1 | Hs.153479 | D79987 |
| 20 | KIF23 | Hs.270845 | NM_004856 |
| 21 | SMC1A | Hs.211602 | D80000 |
| 22 | HEATR2 | Hs.535896 | NM_017802 |
| 23 | MSMB | Hs.255462 | NM_002443 |
| 24 | GCN1L1 | Hs.298716 | D86973 |
| 25 | LRIG1 | Hs.518055 | AB050468 |
| 26 | XPOT | Hs.85951 | AI984005 |
| 27 | PIGV | Hs.259605 | NM_017837 |
| 28 | TMPO | Hs.11355 | AW272611 |
| 29 | UBAP2L | Hs.490551 | NM_014847 |
| 30 | FBXO5 | Hs.520506 | NM_012177 |
| 31 | GPSM2 | Hs.584901 | AW195581 |
| 32 | SLC38A7 | Hs.10499 | NM_018231 |
| 33 | PIR | Hs.495728 | NM_003662 |
| 34 | C11orf60 | Hs.533738 | NM_020153 |
| 35 | NEK2 | Hs.153704 | NM_002497 |
| 36 | NEIL3 | Hs.405467 | NM_018248 |
| 37 | WHSC1 | Hs.113876 | AF083389 |
| 38 | EPN3 | Hs.670090 | NM_017957 |
| 39 | MSMB | Hs.255462 | U22178 |
| 40 | ATP2A2 | Hs.506759 | M23114 |
| 41 | ECT2 | Hs.518299 | NM_018098 |

(continued)

| Number | Gene symbol | UniGene ID | GenBank accession number |
|---|---|---|---|
| 42 | GINS3 | Hs.47125 | AI421812 |
| 43 | AHCYL1 | Hs.705418 | AA479488 |
| 44 | TPI1 | Hs.524219 | NM_000365 |
| 45 | TUBA1B | Hs.719075 | BC006481 |
| 46 | CIRBP | Hs.634522 | NM_001280 |
| 47 | SP100 | Hs.369056 | NM_003113 |
| 48 | TUBB3 | Hs.511743 | NM_006086 |
| 49 | SFRS2B | Hs.476680 | AI343248 |
| 50 | EPHX2 | Hs.212088 | AF233336 |

Table 2-2

| Number | Gene symbol | UniGene ID | GenBank accession number |
|---|---|---|---|
| 51 | TUBA1B | Hs.719075 | BC006379 |
| 52 | TUBA1C | Hs.719091 | BC004949 |
| 53 | TUBA1B | Hs.719075 | BE300252 |
| 54 | EEF1A2 | Hs.433839 | NM_001958 |
| 55 | SDHD | Hs.719164 | NM_003002 |
| 56 | TUBA1B | Hs.719075 | NM_006082 |
| 57 | SLC36A1 | Hs.269004 | AW058600 |
| 58 | DDX41 | Hs.484288 | NM_016222 |
| 59 | EEF1A1 | --- | NM_001403 |
| 60 | PPFIA1 | Hs.530749 | AA195259 |
| 61 | GPRC5A | Hs.631733 | NM_003979 |
| 62 | NCKIPSD | Hs.655006 | NM_016453 |
| 63 | KIF18B | Hs.135094 | AA292789 |
| 64 | IDH3A | Hs.591110 | AI826060 |
| 65 | MAD2L1 | Hs.591697 | NM_002358 |
| 66 | ACTL6A | Hs.435326 | NM_004301 |
| 67 | EEF1A1 | Hs.520703 | NM_001402 |
| 68 | APOM | Hs.534468 | NM_019101 |
| 69 | CDKN3 | Hs.84113 | AF213033 |
| 70 | U2AF2 | Hs.528007 | NM_007279 |
| 71 | CCT2 | Hs.189772 | NM_006431 |
| 72 | JMJD6 | Hs.514505 | AK021780 |
| 73 | MELK | Hs.184339 | NM_014791 |
| 74 | FBN2 | Hs.519294 | NM_001999 |
| 75 | TXNRD1 | Hs.708065 | NM_003330 |

(continued)

| Number | Gene symbol | UniGene ID | GenBank accession number |
|--------|-------------|------------|--------------------------|
| 76 | DYRK2 | Hs.173135 | AI216690 |
| 77 | ESPL1 | Hs.153479 | NM_012291 |
| 78 | TAF2 | Hs.122752 | AK001618 |
| 79 | THYN1 | Hs.13645 | NM_014174 |
| 80 | --- | --- | AL031313 |
| 81 | PRC1 | Hs.567385 | NM_003981 |
| 82 | ZWINT | Hs.591363 | NM_007057 |
| 83 | KIF4A | Hs.648326 | NM_012310 |
| 84 | NUP62 | Hs.574492 | NM_016553 |
| 85 | TPI1 | Hs.524219 | BF116254 |
| 86 | FAM129A | Hs.518662 | NM_022083 |
| 87 | CTTN | Hs.596164 | AU155105 |
| 88 | FAM129A | Hs.518662 | AF288391 |
| 89 | TROAP | Hs.524399 | NM_005480 |
| 90 | GTF3C1 | Hs.371718 | U02619 |
| 91 | LOC91316 | Hs.148656 | AA398569 |
| 92 | ST8SIA2 | Hs.302341 | NM_006011 |
| 93 | FBXW4 | Hs.500822 | AF281859 |
| 94 | CRIM1 | Hs.699247 | BG546884 |
| 95 | IGL@ | Hs.449585 | AJ249377 |

15. A device for determining prognosis of ER-positive breast cancer, the device comprising
a processor, and a computer containing a memory under control of the processor,
wherein the memory is recorded with a computer program for causing the computer to execute the steps of:

acquiring information on expression levels of a first gene group, said gene group consisting of IL6ST, NPY1R, ELOVL5, OPA1, ASAH1, ALDH6A1 and SYBU, in an RNA sample prepared from a biological sample collected from a subject;
determining prognosis of ER-positive breast cancer based on the sum of weighted standardized expression levels of said gene group, wherein each said standardized expression level is the measured gene expression level corrected by a mean gene expression level of RNA samples derived from a plurality of subjects; and
outputting a determination result.

**Patentansprüche**

1. Verfahren zum Erfassen von Informationen in Bezug auf die Prognose von Estrogenrezeptor(ER)-positivem Brustkrebs, wobei das Verfahren umfasst:

einen Messschritt zum Messen der Expressionspegel einer ersten Gengruppe, wobei die Gengruppe aus IL6ST, NPY1R, ELOVL5, OPA1, ASAH1, ALDH6A1 und SYBU besteht, in einer RNA-Probe, die aus einer von einem Subjekt entnommenen biologischen Probe erzeugt worden ist;
einen Analyseschritt zum Analysieren der gemessenen Expressionspegel von Genen; und
einen Erfassungsschritt zum Erfassen von Informationen in Bezug auf die Prognose von ER-positivem Brustkrebs basierend auf der Summe der gewichteten standardisierten Expressionspegel der Gengruppe, wobei jeder standardisierte Expressionspegel der gemessene Genexpressionspegel ist, korrigiert durch einen mittleren Genexpressionspegel von RNA-Proben, abgeleitet aus mehreren Subjekten.

2. Verfahren nach Anspruch 1, wobei die mehreren Subjekte ER-positive Brustkrebspatientinnen sind.

3. Verfahren nach Anspruch 1 oder 2, wobei in dem Messschritt die Expressionspegel unter Einsatz eines Mikroarray gemessen werden.

4. Verfahren nach Anspruch 1 oder 2, wobei in dem Messschritt ferner ein Expressionspegel von mindestens einem Gen gemessen wird, ausgewählt aus CALB2, ASAP2, RAB5C, PTP4A2, ABCC10, OXTR, HSPA2, SMURF2, SLC7A8, RALA, ADRA2A, MYCBP, CX3CR1, ERCC1, DNAJA3, N1NJ1, C4orf43, IFI35, ZNF688, SNX1, CREBL2, HPN, NME3, STS, KLF7, PDHB, NKX3-1, DEXI, GSTM3 und LCMT1.

5. Verfahren nach Anspruch 4, wobei in dem Messschritt die Expressionspegel der Gene unter Einsatz eines Mikroarrays gemessen werden.

6. Verfahren nach einem der Ansprüche 1 bis 5, wobei die Informationen in Bezug auf die Prognose von Brustkrebs Informationen über das Rezidivrisiko nach der Operation sind.

7. Verfahren nach Anspruch 6, wobei die Informationen in Bezug auf das Rezidivrisiko nach der Operation Informationen in Bezug auf das Risiko eines späten Rezidivs nach der Operation sind.

8. Verfahren nach Anspruch 7, wobei die Informationen in Bezug auf das Risiko eines späten Rezidivs Informationen in Bezug auf das Rezidivrisiko über 5 Jahre bis zu 10 Jahre nach der Operation sind.

9. Verfahren nach einem der Ansprüche 1 bis 8, wobei in dem Analyseschritt, der Expressionspegel unter Einsatz eines Klassifizierungsverfahrens, einer hierarchischen Clusteranalyse oder eines Scoringverfahrens analysiert wird.

10. Verfahren nach Anspruch 9, wobei das Klassifizierungsverfahren eine diagonale lineare Diskriminanzanalyse oder eine Analyse im Vergleich zwischen Gruppen ist.

11. Verfahren nach einem der Ansprüche 1 bis 9, wobei in dem Analyseschritt, durch Verwendung des Expressionspegels und einer Diskriminante, die durch die folgende Formel (I) dargestellt wird, Lösung D der Diskriminante bestimmt wird und
im Erfassungsschritt das Risiko eines späten Rezidivs des Subjekts als hoch bestimmt wird, wenn die Lösung D ein positiver Wert ist, und das Risiko eines späten Rezidivs des Subjekts als niedrig bestimmt wird, wenn die Lösung D Null oder ein negativer Wert ist.

$$D = \Sigma_i(w_i \times y_i) - (-4.763756453) \cdots (I)$$

wobei i eine Zahl darstellt, die jedem in Tabelle 1-1 und Tabelle 1-2 gezeigten Gen zugeordnet ist, $w_i$ einen Gewichtungsfaktor des in Tabelle 1-1 und Tabelle 1-2 gezeigten Gens mit der Nummer i darstellt, $y_i$ einen standardisierten Expressionspegel des Gens gemäß einer Formel darstellt, die durch die Formel (II) dargestellt wird:

$$y_i = x_i - m_i \cdots (II)$$

wobei $x_i$ einen Expressionspegel des in Tabelle 1-1 und Tabelle 1-2 gezeigten Gens mit der Nummer i darstellt und $m_i$ einen Mittelwert der Expressionspegel der in Tabelle 1-1 und Tabelle 1-2 gezeigten Gene mit der Nummer i in mehreren Proben darstellt,
und $\Sigma_i$ eine Gesamtsumme der Gene repräsentiert.

Tabelle 1-1

| Gennummer | Gensymbol | Gewichtungsfaktor |
|---|---|---|
| 1 | IL6ST | 1.43235997 |

(fortgesetzt)

| Gennummer | Gensymbol | Gewichtungsfaktor |
|-----------|-----------|-------------------|
| 2 | NPYIR | 0.450736337 |
| 3 | ELOVL5 | 1.793041948 |
| 4 | OPA1 | -2.545950461 |
| 5 | IL6ST | 1.539087017 |
| 6 | ASAH1 | 1.539921375 |
| 7 | IL6ST | 1.151743979 |
| | | |
| 8 | ALDH6A1 | 1.837908119 |
| 9 | SYBU | 1.149172541 |

Tabelle 1-2

| Gennummer | Gensymbol | Gewichtungsfaktor |
|-----------|-----------|-------------------|
| 10 | CALB2 | -1.179415269 |
| 11 | ASAP2 | -2.323532179 |
| 12 | RAB5C | 2.316847761 |
| 13 | PTP4A2 | 2.002072892 |
| 14 | ABCC10 | -2.306701292 |
| 15 | OXTR | -1.1298499 |
| 16 | HSPA2 | 0.964294299 |
| 17 | SMURF2 | -2.109236485 |
| 18 | SLC7A8 | 1.363057605 |
| 19 | RALA | -2.288374092 |
| 20 | ADRA2A | 1.377444437 |
| 21 | MYCBP | 1.688897659 |
| 22 | CX3CR1 | 0.740592577 |
| 23 | ERCC1 | 1.658860215 |
| 24 | DNAJA3 | 2.534304027 |
| 25 | IL6ST | 0.971312607 |
| 26 | NINJ1 | 1.889380552 |
| 27 | C4orf43 | 1.429869188 |
| 28 | IFI35 | 1.323441517 |
| 29 | ZNF688 | 2.158913805 |
| 30 | SNX1 | 2.2492475 |
| 31 | CREBL2 | 1.921069642 |
| 32 | HPN | 1.115597062 |
| 33 | NME3 | 1.661682832 |
| 34 | STS | -1.392023997 |

(fortgesetzt)

| Gennummer | Gensymbol | Gewichtungsfaktor |
|---|---|---|
| 35 | KLF7 | -1.662199312 |
| 36 | PDHB | 2.071027409 |
| 37 | IL6ST | 0.825924727 |
| 38 | NKX3-1 | 0.611126615 |
| 39 | DEXI | 1.847912036 |
| 40 | ASAH1 | 1.575733773 |
| 41 | GSTM3 | 0.648154701 |
| 42 | LCMT1 | 1.7259299 |

**12.** Verfahren nach einem der Ansprüche 1 bis 11, wobei das Subjekt ein Subjekt ist, bei dem 5 Jahre nach der Operation kein Rezidiv von Brustkrebs aufgetreten ist.

**13.** Verfahren nach einem der Ansprüche 1 bis 12, wobei das Subjekt ein Subjekt ist, dessen Risiko eines frühen Rezidivs von Brustkrebs als niedrig bestimmt worden ist.

**14.** Verfahren nach Anspruch 13, wobei das Subjekt ein Subjekt ist, dessen Risiko eines frühen Rezidivs von Brustkrebs basierend auf unter Einsatz eines Mikroarrays, umfassend für die in Tabelle 2-1 und Tabelle 2-2 gezeigten 95 Gene spezifische Sondensätze, gemessenen Genexpressionspegeln als niedrig bestimmt worden ist.

Tabelle 2-1

| Nummer | Gensymbol | UniGene-ID | GenBank-Zugangsnummer |
|---|---|---|---|
| 1 | KIF15 | Hs.646856 | NM 020242 |
| 2 | DTL | Hs.656473 | NM 016448 |
| 3 | DONSON | Hs.436341 | AF232674 |
| 4 | KPNA2 | Hs. 594238 | NM 002266 |
| 5 | PNRC1 | Hs.75969 | AF279899 |
| 6 | MED14 | Hs.407604 | AF135802 |
| 7 | KLC2 | Hs.280792 | NM_022822 |
| 8 | JMJD6 | Hs.514505 | AK021780 |
| 9 | LRRC59 | Hs.370927 | AK025328 |
| 10 | CAND1 | Hs.546407 | AB020636 |
| 11 | NUSAP1 | Hs.615092 | NM 016359 |
| 12 | CCBL2 | Hs.481898 | BC000819 |
| 13 | KIAA0406 | Hs.655481 | AB007866 |
| 14 | PLOD2 | Hs.477866 | NM_000935 |
| 15 | CTTN | Hs.596164 | NM_005231 |
| 16 | HSPB1 | Hs.520973 | NM_001540 |
| 17 | BUB1B | Hs.631699 | NM_001211 |
| 18 | TUBAIC | Hs.719091 | BC005946 |
| 19 | ESPL1 | Hs.13479 | D79987 |
| 20 | KIF23 | Hs.270845 | NM_004856 |

(fortgesetzt)

| Nummer | Gensymbol | UniGene-ID | GenBank-Zugangsnummer |
|---|---|---|---|
| 21 | SMC1A | Hs.211602 | D80000 |
| 22 | HEATR2 | Hs.535896 | NM_017805 |
| 23 | MSMB | Hs.255462 | NM_002443 |
| 24 | GCNIL1 | Hs.298716 | D86973 |
| 25 | LRIG1 | Hs.518055 | AB050468 |
| 26 | XPOT | Hs.85951 | AI984005 |
| 27 | PIGV | Hs.259605 | NM_017837 |
| 28 | TMPO | Hs.11355 | AW275611 |
| 29 | UBAP2L | Hs.490551 | NM_014847 |
| 30 | FBXO5 | Hs.520506 | NM_012177 |
| 31 | GPSM2 | Hs.584901 | AW195581 |
| 32 | SLC38A7 | Hs.10499 | NM_918231 |
| 33 | PIR | Hs.495728 | NM_003662 |
| 34 | C11orf60 | Hs.533738 | NM_020153 |
| 35 | NEK2 | HS.153704 | NM_002497 |
| 36 | NEIL3 | Hs.405467 | NM_018248 |
| 37 | WHSC1 | Hs.113876 | AF083389 |
| 38 | EPN3 | Hs.670090 | NM_017957 |
| 39 | MSMB | Hs.255462 | U22178 |
| 40 | ATP2A2 | Hs.506759 | M23114 |
| 41 | ECT2 | Hs.519299 | NM_018098 |
| 42 | GINS3 | Hs.47125 | AI421812 |
| 43 | AHCYL1 | Hs.705418 | AA479488 |
| 44 | TPI1 | Hs.524219 | NM_000365 |
| 45 | TUBA1B | Hs.719075 | BC006481 |
| 46 | CIRBP | Hs.634522 | NM_001280 |
| 47 | SP100 | Hs.369056 | NM_003113 |
| 48 | TUBB3 | Hs.511743 | NM_006096 |
| 49 | SFRS2B | Hs.476680 | AI343248 |
| 50 | EPHX2 | Hs.212088 | AF233336 |

Tabelle 2-2

| Nummer | Gensymbol | UniGene-ID | GenBank-Zugangsnummer |
|---|---|---|---|
| 51 | TUBA1B | Hs.719075 | BC006379 |
| 52 | TUBA1C | Hs.719091 | BC004949 |
| 53 | TUBA1B | Hs.719075 | BE300252 |
| 54 | EEFIA2 | Hs.433839 | NM_001958 |

(fortgesetzt)

| Nummer | Gensymbol | UniGene-ID | GenBank-Zugangsnummer |
|---|---|---|---|
| 55 | SDHD | Hs.719164 | NM_003002 |
| 56 | TUBA1B | Hs.719075 | NM_906082 |
| 57 | SLC36A1 | Hs.269004 | AW058600 |
| 58 | DDX41 | Hs.484288 | NM_016222 |
| 59 | EEFIA1 | --- | NM_001403 |
| 60 | PPFIA1 | Hs.530749 | AA195259 |
| 61 | GPRC5A | Hs.631733 | NM_003979 |
| 62 | NCKIPSD | Hs.655006 | NM_016453 |
| 63 | KIFI8B | Hs.135094 | AA292789 |
| 64 | IDH3A | Hs.591110 | A1826060 |
| 65 | MAD2L1 | Hs.591697 | NM_002358 |
| 66 | ACTL6A | Hs.435326 | NM_004301 |
| 67 | FEFIA1 | Hs.520703 | NM_001402 |
| 68 | APOM | Hs.534468 | NM_019101 |
| 69 | CDKN3 | Hs.84113 | AF213033 |
| 70 | U2AF2 | Hs.528007 | NM_007279 |
| 71 | CCT2 | Hs.189772 | NM_006431 |
| 72 | JMJD6 | Hs.514505 | AK021780 |
| 73 | MELK | Hs.184339 | NM_014791 |
| 74 | FBN2 | Hs.519294 | NM_001999 |
| 75 | TXNRD1 | Hs.708065 | NM_003330 |
| 76 | DYRK2 | Hs.173135 | AI216690 |
| 77 | ESPL1 | Hs.153479 | NM_012291 |
| 78 | TAF2 | HS.122752 | AK001618 |
| 79 | THYN1 | Hs. 13645 | NM_014174 |
| 80 | --- | --- | AL031313 |
| 81 | PRC1 | Hs.567385 | NM_003981 |
| 82 | ZWINT | Hs.591363 | NM_007057 |
| 83 | KIF4A | Hs.648326 | M_012310 |
| 84 | NUP62 | Hs.574492 | NM_016553 |
| 85 | TPI1 | Hs.524219 | BP116254 |
| 86 | FAM129A | Hs.518662 | NM_022083 |
| 87 | CTTN | Hs.596164 | AU155105 |
| 88 | FAM129A | Hs.518662 | AF288391 |
| 89 | TROAP | Hs.5241199 | NM_005480 |
| 90 | GTF3C1 | Hs.371718 | U02619 |
| 91 | LOC91316 | Hs.148656 | AA398569 |
| 92 | ST8SIA2 | Hs.302341 | NM_006011 |

(fortgesetzt)

| Nummer | Gensymbol | UniGene-ID | GenBank-Zugangsnummer |
|---|---|---|---|
| 93 | FBXW4 | Hs.500822 | AF281859 |
| 94 | CRIM1 | Hs.699247 | BG546884 |
| 95 | IGL@ | Hs.449585 | AJ249377 |

15. Vorrichtung zum Bestimmen einer Prognose von ER-positivem Brustkrebs, wobei die Vorrichtung einen Prozessor und einen Computer mit einem durch den Prozessor gesteuerten Speicher umfasst, wobei der Speicher mit einem Computerprogramm aufgezeichnet wird, um zu bewirken, dass der Computer die folgenden Schritte ausführt:

Erfassen von Informationen in Bezug auf Expressionspegel einer ersten Gengruppe, wobei die Gengruppe aus IL6ST, NPY1R, ELOVL5, OPA1, ASAH1, ALDH6A1 und SYBU besteht, in einer RNA-Probe, die aus einer von einem Subjekt entnommenen biologischen Probe erzeugt worden ist;
Bestimmen einer Prognose von ER-positivem Brustkrebs basierend auf der Summe der gewichteten standardisierten Expressionspegel der Gengruppe, wobei jeder standardisierte Expressionspegel der gemessene Genexpressionspegel ist, korrigiert durch einen mittleren Genexpressionspegel von RNA-Proben, abgeleitet aus mehreren Subjekten; und
Ausgeben eines Bestimmungsergebnisses.

## Revendications

1. Procédé destiné à acquérir des informations sur le pronostic d'un cancer du sein positif au récepteur d'œstrogènes (RE), le procédé comprenant :

une étape de mesure consistant à mesurer des niveaux d'expression d'un premier groupe de gènes, ledit groupe de gènes étant constitué par les IL6ST, NPY1R, ELOVL5, OPA1, ASAH1, ALDH6A1 et SYBU, dans un échantillon d'ARN préparé à partir d'un échantillon biologique recueilli d'un sujet ;
une étape d'analyse consistant à analyser les niveaux mesurés d'expression des gènes ; et
une étape d'acquisition consistant à acquérir des informations sur le pronostic d'un cancer du sein positif au RE sur la base de la somme des niveaux d'expression normalisés et pondérés dudit groupe de gènes, dans laquelle chaque dit niveau d'expression normalisé est le niveau d'expression génique mesuré corrigé par un niveau d'expression génique moyen d'échantillons d'ARN dérivés d'une pluralité de sujets.

2. Procédé de la revendication 1, dans lequel ladite pluralité de sujets est composée de patients à cancers du sein positifs au RE.

3. Procédé selon les revendications 1 ou 2, dans lequel, dans l'étape de mesure, les niveaux d'expression sont mesurés en utilisant un micro-arrangement.

4. Procédé selon les revendications 1 ou 2, dans lequel, dans l'étape de mesure, on mesure en outre un niveau d'expression d'au moins un gène choisi parmi les CALB2, ASAP2, RAB5C, PTP4A2, ABCC10, OXTR, HSPA2, SMURF2, SLC7A8, RALA, ADRA2A, MYCBP, CX3CR1, ERCC1, DNAJA3, NINJ1 C4orf43, IFI35, ZNF688, SNX1 CREBL2, HPN, NME3, STS, KLF7, PDHB, NKX3-1, DEXI, GSTM3 et LCMT1.

5. Procédé selon la revendication 4, dans lequel, dans l'étape de mesure, les niveaux d'expression desdits gènes sont mesurés en utilisant un micro-arrangement.

6. Procédé selon l'une quelconque des revendications 1 à 5, dans lequel les informations sur le pronostic d'un cancer du sein sont des informations sur le risque de récurrence après une chirurgie.

7. Procédé selon la revendication 6, dans lequel les informations sur le risque de récurrence après une chirurgie sont des informations sur le risque de récurrence tardive après une chirurgie.

8. Procédé selon la revendication 7, dans lequel les informations sur le risque de récurrence tardive sont des informations sur le risque de récurrence au-delà de 5 ans dans les 10 ans après une chirurgie.

9. Procédé selon l'une quelconque des revendications 1 à 8, dans lequel, dans l'étape d'analyse, le niveau d'expression est analysé en utilisant une méthode de classification, une analyse à classification hiérarchique par groupes ou une méthode de notation.

10. Procédé selon la revendication 9, dans lequel la méthode de classification est une analyse discriminante linéaire diagonale ou une analyse entre groupes.

11. Procédé selon l'une quelconque des revendications 1 à 9, dans lequel, dans l'étape d'analyse, en utilisant le niveau d'expression et un discriminant représenté par la formule (I) suivante, on détermine une solution D du discriminant et, dans l'étape d'acquisition, on détermine que le risque de récurrence tardive du sujet est élevé quand la solution D est une valeur positive et que le risque de récurrence tardive du sujet est faible quand la solution D est égale à zéro ou est une valeur négative.

$$D = \Sigma_i(w_i \ x \ y_i) - (-4,763756453)...(I)$$

dans laquelle i représente un nombre attribué à chaque gène présenté dans le tableau 1-1 et le tableau 1-2, $w_i$ représente un facteur de pondération d'un gène numéroté i présenté dans le tableau 1-1 et le tableau 1-2, $y_i$ représente un niveau d'expression normalisé d'un gène selon une formule représentée par la formule (II) :

$$y_i = x_i - m_i...(II)$$

dans laquelle $x_i$ représente un niveau d'expression d'un gène numéroté i présenté dans le tableau 1-1 et le tableau 1-2 et $m_i$ représente une valeur moyenne des niveaux d'expression de gènes numérotés i, présentés dans le tableau 1-1 et le tableau 1-2, sur les spécimens,
et $\Sigma_i$ représente une somme totale sur les gènes.

Tableau 1-1

| Numéro du gène | Symbole du gène | Facteur de pondération |
|---|---|---|
| 1 | IL6ST | 1,43235997 |
| 2 | NPY1R | 0,450736337 |
| 3 | ELOVL5 | 1,793041948 |
| 4 | OPA1 | -2,545950461 |
| 5 | IL6ST | 1,539087017 |
| 6 | AsAH1 | 1,539921375 |
| 7 | IL6ST | 1,151143919 |
| 8 | ALDH6A1 | 1,837908119 |
| 9 | SYBU | 1,149172541 |

Tableau 1-2

| Numéro du gène | Symbole du gène | Facteur de pondération |
|---|---|---|
| 10 | CALB2 | -1,179415269 |
| 11 | ASAP2 | -2,323532179 |
| 12 | RAB5C | 2,316947761 |
| 13 | PTP4A2 | 2,002072892 |

(suite)

| Numéro du gène | Symbole du gène | Facteur de pondération |
|---|---|---|
| 14 | ABCC10 | -2,306701292 |
| 15 | OXTR | -1,1298499 |
| 16 | HSPA2 | 0,964294299 |
| 17 | SMURF2 | -2,109236495 |
| 18 | SLC7A8 | 1,363057605 |
| 19 | RALA | -2,288374092 |
| 20 | ADRA2A | 1,377444437 |
| 21 | MYCBP | 1,688897659 |
| 22 | CX3CR1 | 0,740592577 |
| 23 | ERCC1 | 1,658860215 |
| 24 | DNAJA3 | 2,534304027 |
| 25 | IL6ST | 0,971312607 |
| 26 | NINJI | 1,889380552 |
| 27 | C4orf43 | 1,429869188 |
| 28 | IFI35 | 1,323441517 |
| 29 | ZNF688 | 2,158913805 |
| 30 | SNX1 | 2,2492475 |
| 31 | CREBL2 | 1,921069642 |
| 32 | HPN | 1,115597062 |
| 33 | NME3 | 1,061682832 |
| 34 | STS | -1,392023997 |
| 35 | KLF7 | -1,662199312 |
| 36 | PDHB | 2,071027409 |
| 37 | IL6ST | 0,825924727 |
| 38 | NKX3- | 0,611126615 |
| 39 | DEXI | 1,847912036 |
| 40 | ASAH | 1,575733773 |
| 41 | GSTM3 | 0,648154701 |
| 42 | LCMT1 | 1,7259299 |

12. Procédé selon l'une quelconque des revendications 1 à 11, dans lequel le sujet est un sujet qui n'avait pas de récurrence d'un cancer du sein pendant 5 ans après une chirurgie.

13. Procédé selon l'une quelconque des revendications 1 à 12, dans lequel le sujet est un sujet chez qui on a déterminé que le risque de récurrence précoce d'un cancer du sein était faible.

14. Procédé selon la revendication 13, dans lequel le sujet est un sujet chez qui on a déterminé que le risque de récurrence précoce d'un cancer du sein était faible, sur la base des niveaux d'expression génique mesurés en utilisant un micro-arrangement comprenant des ensembles de sondes spécifiques des 95 gènes présentés dans le tableau 2-1 et le tableau 2-2.

Tableau 2-1

| Numéro | Symbole du gène | ID d'UniGène | Numéro d'accès GenBank |
|---|---|---|---|
| 1 | KIF15 | Hs.646856 | NM_020242 |
| 2 | DTL | Hs.656473 | NM_016448 |
| 3 | DONSON | Hs.436341 | AF232674 |
| 4 | KPNA2 | Hs.594238 | NM_002266 |
| 5 | PNRC1 | Hs.75969 | AF279899 |
| 6 | MED14 | Hs.407604 | AF135802 |
| 7 | KLC2 | Hs.280792 | NM_022822 |
| 8 | JMJD6 | Hs.514505 | AK021780 |
| 9 | LRRC59 | Hs.370927 | AK025328 |
| 10 | CANDI | Hs.546407 | AB020636 |
| 11 | NUSAP1 | Hs.615092 | NM_016359 |
| 12 | CCBL2 | Hs.481898 | BC000819 |
| 13 | KIAA0406 | Hs.655481 | AB007866 |
| 14 | PLOD2 | Hs.477866 | NM_0000935 |
| 15 | CTTN | Hs.596164 | NM_005231 |
| 16 | HSPB1 | Hs.520973 | NM_001540 |
| 17 | BUB1B | Hs.631699 | NM_001211 |
| 18 | TUBA1C | Hs.719091 | BC005946 |
| 19 | ESPL1 | Hs.153479 | D79987 |
| 20 | KIF23 | Hs.270845 | NM_004856 |
| 2 | SMCIA | Hs.211602 | D80000 |
| 22 | HEATR2 | Hs.535896 | NM_017802 |
| 23 | MSMB | Hs.255462 | NM_002443 |
| 24 | GCNIL1 | Hs.298716 | D86973 |
| 25 | LRIG1 | Hs.518055 | AB050468 |
| 26 | XPOT | Hs.85951 | A1984005 |
| 27 | PIGV | Hs.259605 | NM_017837 |
| 28 | TMPO | Hs.11355 | AW272611 |
| | | | |
| 29 | UBAP2L | Hs.490551 | NM_014847 |
| 30 | FBX05 | Hs.520506 | NM_012177 |
| 31 | GPSM2 | Hs.584901 | AW195581 |
| 32 | SLC38A7 | Hs.10499 | NM_018231 |
| 33 | PIR | Hs.495728 | NM_003662 |
| 34 | C11orf60 | Hs.533738 | NM_020153 |
| 35 | NEK2 | Hs.153704 | NM_002497 |
| 36 | NBIL3 | Hs.405467 | NM_018248 |

(suite)

| Numéro | Symbole du gène | ID d'UniGène | Numéro d'accès GenBank |
|---|---|---|---|
| 37 | WHSC1 | Hs.113876 | AF083389 |
| 38 | EPN3 | Hs.670090 | NM_017957 |
| 39 | MSMB | Hs.255462 | U22178 |
| 40 | ATP2A2 | Hs.506759 | M23114 |
| 41 | ECT2 | Hs.518299 | NM_018098 |
| 43 | GINS3 | Hs.47125 | AI421812 |
| 43 | AHCYL1 | Hs.703418 | AA479488 |
| 44 | TPI1 | Hs.524219 | NM_000365 |
| 45 | TUBA1B | Hs.719075 | BC006481 |
| 46 | CIRBP | Hs.634522 | NM_001280 |
| 47 | SP100 | Hs.369056 | NM_003113 |
| 48 | TUBB3 | Hs.511743 | NM_006086 |
| 49 | SFRS2B | Hs.476680 | A1343248 |
| 50 | EPHX2 | Hs.212088 | AF233336 |

Tableau 2-2

| Numéro | Symbole du gène | ID d'UniGène | Numéro d'accès GenBank |
|---|---|---|---|
| 51 | TUBA1B | Hs.719075 | BC006379 |
| 52 | TUBA1C | Hs.719091 | BC004949 |
| 53 | TUBA1B | Hs.719075 | BE300252 |
| 54 | EEFIA2 | Hs.433839 | NM_001958 |
| 55 | SDHD | Hs.719164 | NM_003002 |
| 56 | TUBA1B | Hs.719075 | NM_006082 |
| 51 | SLC36A1 | Hs.269004 | AW058600 |
| 58 | DDX41 | Hs.484288 | NM_016222 |
| 59 | EEFIA1 | --- | NM_001403 |
| 60 | PPFIA1 | Hs.530749 | AA195259 |
| 61 | GPRC5A | Hs.631733 | NM_003979 |
| 62 | NCKIPSD | Hs.655006 | NM_016453 |
| 63 | KIF18B | Hs.135094 | AA292789 |
| 64 | IDH3A | Hs.591110 | A1826060 |
| 65 | MAD2L1 | Hs.591697 | NM_002358 |
| 66 | ACTL6A | Hs.435326 | 004301 |
| 67 | EEF1A1 | Hs.520703 | NM_001402 |
| 68 | APOM | Hs.534468 | NM_019101 |
| 69 | CDKN3 | Hs.84113 | AF213033 |
| 70 | U2AF2 | Hs.528007 | NM_007279 |

(suite)

| Numéro | Symbole du gène | ID d'UniGène | Numéro d'accès GenBank |
|--------|-----------------|--------------|------------------------|
| 71 | CCT2 | Hs.189772 | NM_006431 |
| 72 | JMJD6 | Hs.514505 | AK021780 |
| 73 | MELK | Hs.184339 | NM_014791 |
| 74 | FBN2 | Hs.519294 | NM_001999 |
| 75 | TXNRD1 | Hs.708065 | NM_003330 |
| 76 | DYRK2 | Hs.173135 | A1216690 |
| 77 | ESPL1 | Hs.153479 | NM_012291 |
| 78 | TAF2 | Hs.122752 | AK001618 |
|  |  |  |  |
| 79 | THYN1 | Hs.13645 | NM_014174 |
| 80 | --- | --- | AL031313 |
| 81 | PRC1 | Hs.567385 | NM_003981 |
| 82 | ZWINT | Hs.591363 | NM_007057 |
| 83 | KIF4A | Hs.648326 | HM_012310 |
| 84 | NUP62 | Hs.574492 | NM_016553 |
| 85 | TPI1 | Hs.524219 | BFI16254 |
| 86 | FAM129A | Hs.518662 | NM_022083 |
| 87 | CTTN | Hs.596164 | AU155105 |
| 88 | FAM129A | Hs.518662 | AF288391 |
| 89 | TROAP | Hs.524399 | NM_005480 |
| 90 | GTF3C1 | Hs.371718 | U02619 |
| 91 | LOC91316 | Hs.148656 | AA398569 |
| 92 | ST8SIA2 | Hs.302341 | NM_006011 |
| 93 | FBXW4 | Hs.500822 | AF281859 |
| 94 | CRIM1 | Hs.699247 | BG546884 |
| 95 | IGL@ | Hs.449585 | AJ249377 |

15. Dispositif destiné à déterminer le pronostic d'un cancer du sein positif au RE, le dispositif comprenant un processeur et un ordinateur contenant une mémoire sous le contrôle du processeur, dans lequel la mémoire est enregistrée avec un programme informatique destiné à faire que l'ordinateur exécute les étapes :

d'acquisition d'informations sur les niveaux d'expression d'un premier groupe de gènes, ledit groupe de gènes étant constitué par les IL6ST, NPY1R, ELOVL5, OPA1, ASAH1, ALDH6A1 et SYBU, dans un échantillon d'ARN préparé à partir d'un échantillon biologique recueilli d'un sujet ;
de détermination du pronostic d'un cancer du sein positif au RE sur la base de la somme des niveaux d'expression normalisés et pondérés dudit groupe de gènes, dans laquelle chaque dit niveau d'expression normalisé est le niveau d'expression génique mesuré corrigé par un niveau d'expression génique moyen d'échantillons d'ARN dérivés d'une pluralité de sujets ; et
d'obtention d'un résultat de détermination.

FIG. 1

# FIG. 2

# FIG. 3

## FIG. 4

VAB — AFFYMETRIX U133 PLUS 2.0

S4-1 → Surgery pN1
Oncotype DX®

S4-2 → Low risk → 42GC
- S4-3 → Low risk → 5y-HT
- S4-4 → High risk → 10y-HT

S4-5 → High risk → CT / 155GC
- S4-6 → L-CS* / 42GC
  - S4-7 → Low risk → 5y-HT
  - S4-8 → High risk → 10y-HT
- S4-9 → H-CS* → Additional CT / 42GC
  - S4-10 → Low risk → 5y-HT
  - S4-11 → High risk → 10y-HT

EP 3 553 185 B1

# FIG. 5

# FIG. 6

## FIG. 7

```
        ┌──────────────┐
        │    START     │
        └──────┬───────┘
               │
               ▼
   ┌───────────────────────┐
   │  ACQUIRE FLUORESCENCE │  S101
   │       INTENSITY       │
   └───────────┬───────────┘
               │
               ▼
   ┌───────────────────────┐
   │  ACQUIRE SOLUTION D OF │  S102
   │       FORMULA (I)      │
   └───────────┬───────────┘
               │          S103
               ▼
          ╱─────────╲    NO
         ╱   D > 0 ?   ╲ ─────────────────────────┐
          ╲─────────╱                              │
               │ YES                               │
               ▼                                   ▼
   ┌───────────────────────┐         ┌───────────────────────┐
   │  DETERMINE THAT LATE  │  S104   │  DETERMINE THAT LATE  │  S105
   │ RECURRENCE RISK IS HIGH│        │ RECURRENCE RISK IS LOW │
   └───────────┬───────────┘         └───────────┬───────────┘
               │◄──────────────────────────────────┘
               ▼
   ┌───────────────────────┐
   │  OUTPUT DETERMINATION │  S106
   │        RESULT         │
   └───────────┬───────────┘
               │
               ▼
        ┌──────────────┐
        │     END      │
        └──────────────┘
```

# FIG. 8

## FIG. 9A

## FIG. 9B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110263444

**Non-patent literature cited in the description**

- **SAMBROOK, J. et al.** Molecular Cloning: A Laboratory Manual. Cold Spring Harbor Laboratory Press, 1989
- **CULHANE A.C. et al.** Between-group analysis of microarray data. *Bioinformatics,* 2002, vol. 18, 1600-1608
- **TSUNASHIMA R. et al.** Construction of multi-gene classifier for prediction of response to and prognosis after neoadjuvant chemotherapy for estrogen receptor positive breast cancers. *Cancer Letters,* 2015, vol. 365, 166-173
- **CHOI J.K et al.** Combining multiple microarray studies and modeling interstudy variation. *Bioinformatics,* 2003, vol. 19 (1), i84-94